# EUROPÄISCHE PATENTSCHRIFT

(11) **EP 3 284 517 B1**
(45) Veröffentlichungstag und Bekanntmachung des Hinweises auf die Patenterteilung: **02.10.2019**
(21) Anmeldenummer: 17184617.3
(22) Anmeldetag: 03.08.2017
(51) Int. Cl.: A61N 1/375, A61N 1/05, A61N 1/02

(54) **IMPLANTAT UND VERFAHREN ZUR IDENTIFIKATION EINER ELEKTRODENLEITUNG**
IMPLANT AND METHOD FOR THE IDENTIFICATION OF AN ELECTRODE LEAD
IMPLANT ET PROCÉDÉ D'IDENTIFICATION D'UNE LIGNE D'ÉLECTRODES

(30) Priorität: 16.08.2016 EP 16184299; 16.08.2016 EP 16184298; 16.08.2016 EP 16184297
(43) Veröffentlichungstag der Anmeldung: 21.02.2018
(73) Patentinhaber: BIOTRONIK SE & Co. KG, 12359 Berlin (DE)
(72) Erfinder: RUMP, Jens, 12049 Berlin (DE); WEISS, Ingo, 12435 Berlin (DE)
(74) Vertreter: Keck, Hans-Georg

(56) Entgegenhaltungen:
- DE-A1-102008 040 867
- US-A1- 2003 018 369
- US-A1- 2007 203 547
- US-A1- 2014 343 633

## Beschreibung

Die Erfindung betrifft ein Implantat mit einem hermetisch dicht abgeschlossenem Gehäuse, wobei in dem Gehäuse eine Steuereinrichtung angeordnet ist, und mit einem an dem Gehäuse befestigten Header aufweisend mindestens eine Buchse zur Verbindung mit einem Stecker einer Elektrodenleitung und eine Kommunikationsantenne, welche elektrisch mit der Steuereinrichtung verbunden ist, ein Verfahren zur Identifikation einer Elektrodenleitung sowie eine Elektrodenleitung.

Implantate (implantierbare Medizingeräte, IMD) wie Herzschrittmacher (Pacemaker), Defibrillatoren oder neurologische Geräte wie Himschrittmacher für tiefe Hirnstimulation (Deep Brain Stimulation), Rückenmarkstimulationsgeräte, TENS (Transcutaneous Electrical Nerve Stimulator), Geräte für muskuläre Stimulationstherapie, oder Diagnosegeräte, welche die chemischen Eigenschaften des Blutes des Patienten, anderer Körperteile oder andere Körpereigenschaften und -parameter untersuchen, verwenden häufig Elektrodenleitungen, die in den Körper des Patienten geführt werden und dort zumindest über den Zeitraum der Behandlung oder Messung verbleiben. Die Elektrodenleitungen sind mit dem Implantat elektrisch leitend verbunden.

Die Implantate umfassen üblicherweise ein körperverträgliches Gehäuse mit einer dazugehörigen elektrischen/elektronischen Schaltung und einer Energieversorgung, z. B. einer Batterie. Die elektrische/elektronische Schaltung, eine mit der Schaltung elektrisch verbundene Steuereinrichtung und eine Energieversorgung sind häufig innerhalb des Gehäuses angeordnet, welches hermetisch dicht abgeschlossen ist. Das Implantat besitzt mindestens eine Buchse, an der eine oder mehrere Elektrodenleitungen angeschlossen werden können, beispielsweise mittels eines Steckers. Eine Elektrodenleitung dient der Übertragung der elektrischen Energie (eines elektrischen Potentials) bzw. von Daten vom Gehäuse zu dem behandelten oder untersuchten Körperteil und umgekehrt. Hierfür muss eine elektrische Verbindung zwischen den im Gehäuseinneren angeordneten elektrischen/elektronischen Bauteilen einschließlich Steuereinrichtung und der Elektrodenleitung oder den Elektrodenleitungen hergestellt werden. Diese elektrische Verbindung wird in der Regel mittels eines sogenannten Headers realisiert. Eine mit dem Gehäuse verbundene Durchführung sorgt dabei für mindestens eine elektrische Verbindung zwischen dem Inneren des Gehäuses und dem Äußeren und ist gleichzeitig für den hermetischen Abschluss des Gehäuses verantwortlich. Der über der Durchführung befestigte Header führt die elektrische Verbindung der Durchführung weiter zu der mindestens einen Buchse, die zur elektrischen und mechanischen Verbindung mit einer Elektrodenleitung dient. Header und Durchführung können auch als eine kombinierte Baugruppe eingesetzt werden. Im Folgenden wird eine beliebige Baugruppe des Implantats, welche die elektrische Verbindung der im Innern des hermetisch dicht abgeschlossenen Gehäuses angeordneten Elemente nach Außen herstellt und die mindestens eine Buchse zum Einstecken des mindestens einen Steckers aufweist, als Header bezeichnet.

Im Rahmen der vorliegenden Erfindung wird unter dem Begriff "Elektrodenleitung" eine Leitung mit einem elektrischen Leiter oder mehreren elektrischen Leitern zusammen mit dem umhüllenden, den oder die elektrischen Leiter nach außen und ggf. gegeneinander elektrisch isolierenden Isolationsschlauch sowie allen weiteren funktionellen Elementen, die mit der Leitung fest verbunden sind, verstanden. Die Elektrodenleitung umfasst in der Regel an ihrem distalen Ende Elektroden, die z. B. als Ringe entlang der Elektrodenleitung oder in Form einer Matrix auf einem Patch angeordnet sind. Manche Elektrodenleitungen umfassen an ihrem distalen Ende auch eine sogenannte Elektrodenspitze, mittels der die elektrische Energie von dem oder den Leiter(n) in das zu behandelnde Gewebe eingeleitet wird. Häufig ist eine Elektrodenspitze mit Ankerelementen oder Haltestrukturen versehen, mit denen die Konstanz der räumlichen Lage der Übergangsstelle der elektrischen Energie in das zu behandelnde Gewebe sichergestellt wird. Die Elektrodenspitze kann als Ableit-, Stimulations- oder Messelektrode ausgebildet sein. Zusätzlich oder alternativ zu einer Elektrode oder einer Elektrodenspitze können Elektrodenleitungen auch einen oder mehrere Sensoren zur Erfassung von Parametern aufweisen. Weiter besitzt die Elektrodenleitung in der Regel, beispielsweise an ihrem proximalen Ende, einen Stecker, mit der die Elektrodenleitung mit einem Implantat verbunden werden kann, wobei der Stecker hierfür in eine entsprechende Buchse des Implantats eingesteckt wird. Der Stecker besitzt einen oder mehrere Kontaktelemente (Pole, Anschlüsse), wobei jedes Kontaktelement mit genau einem elektrischen Leiter der Elektrodenleitung verbunden ist. Entsprechend ist in der Buchse für jedes Kontaktelement der Elektrodenleitung ein Kontaktelement der Buchse vorgesehen.

An moderne Implantate, beispielsweise einen Mehrkammer-Herzschrittmacher, einen implantierbaren Kardioverter-Defibrillator (ICD) oder einen Neurostimulator, werden häufig mehrere Elektrodenleitungen angeschlossen. Hierbei besteht das Bestreben, die Elektrodenleitungen und ihre Anschlüsse möglichst dünn bzw. klein zu gestalten. Dies erschwert jedoch die gut sichtbare Markierung der Stecker und Anschlüsse sowie die Unterscheidung der Elektrodenleitungen. Darüber hinaus steigt mit zunehmender Anzahl von Elektrodenleitungen die Gefahr, dass einzelne Elektrodenleitungen verwechselt und/oder falsch angeschlossen werden. Deshalb ist es wünschenswert, wenn ein Implantat erkennt, welche Elektrodenleitungen angeschlossen sind, so dass es diese adäquat ansteuern kann. Zudem ist es zum Betreiben des Implantats hilfreich, wenn die Elektrodenleitungen und/oder ihre Eigenschaften für das Implantat identifizierbar sind.

Dokument US 2004/0073265 A1 beschreibt eine Vorrichtung, die eine Möglichkeit bietet, falsch verbundene Koronarleitungen und/oder falsche Verbindungen zu Herzrhythmusmanagement-Vorrichtungen zu erkennen. Dazu erzeugt eine Spannungseinbringungsvorrichtung eines Schrittmachers einen Spannungspuls zwischen einer Elektrode, die mit einer Leitung mit dem Schrittmacher verbunden ist, und einer Kopf- oder Gehäuseelektrode des Schrittmachers. Die Gehäuseelektrode sendet ein Verbindungssignal. Die Elektrode wird genutzt, um ein korrespondierendes Verbindungssignal unter Nutzung der Leitung zu messen. Ein Messmodul der Vorrichtung misst weiter eine oder mehrere Eigenschaften des korrespondierenden Verbindungssignals wie seine Stromstärke, Spannung, Impedanz und/oder sein Zeitverzug (nach Aussendung des Spannungspulses). Die Signaleigenschaften können von einer oder mehreren Leitungen und/oder vom übermittelnden Gewebe und Flüssigkeiten (beispielsweise einem Herz inklusive einer oder mehrere seiner Kammern), die dazwischen liegen, beeinflusst werden. Ein Vergleichsmodul des Schrittmachers kann daraufhin feststellen, ob die Leitung ordnungsgemäß zu einem Kontakt des Schrittmachers geführt ist, wobei eine oder mehrere Eigenschaften des korrespondierenden Verbindungssignals mit geeigneten vorgewählten Wertebereichen verglichen werden. Beispielsweise kann eine gemessene Impedanz mit einem erwarteten Impedanzbereich verglichen werden. Die in der Druckschrift beschriebene Vorrichtung identifiziert damit nicht selektiv die Leitung, sondern testet vielmehr, ob das korrespondierende Vergleichssignal, das über eine Leitung nach Anregung des Körpers durch einen Spannungspuls einer Gehäuseelektrode des Schrittmachers empfangen wird, Eigenschaften innerhalb eines vorgegebenen Wertebereichs aufweist. Die Eigenschaften des korrespondierenden Vergleichssignals werden auch durch das angeregte Körpergewebe zwischen der Gehäuseelektrode des Schrittmachers und der empfangenden Elektrode bestimmt. Nur sehr grobe Abweichungen, wie sie durch eine nicht verbundene oder vollkommen falsche Art von Leitung auftreten, können sicher auf die Leitung zurückgeführt werden, geringere Abweichungen können körperbedingt sein. Die oben genannte Vorrichtung kann damit die sichere und gezielte Erkennung und Unterscheidung von Elektrodenleitungen mit ähnlichen Eigenschaften nicht gewährleisten.

Eine ähnliche Vorrichtung wird auch in Dokument US 2006/0212083 A1 offenbart. Auch in dieser Druckschrift wird betont, dass die Signaleigenschaften von den Leitungen und/oder von dem/der dazwischen liegenden übermittelnden Gewebe und Flüssigkeit beeinflusst werden.

Dokument US 2011/0112609 A1 beschreibt ein System zur Rückenmarksstimulation mit mindestens einer implantierbaren Stimulationsleitung. Es umfasst insbesondere ein ärztliches Programmiergerät und einen implantierbaren Pulsgenerator, der mit einer oder mehreren implantierbaren Stimulationsleitungen verbunden ist, die je eine Vielzahl von Elektroden tragen. Die Stimulationsleitung weist ein oder zwei Leitungskörper auf. Die Elektroden passen genau in den Epiduralraum der Wirbelsäule. Da das dortige Gewebe leitend ist, können elektrische Messungen zwischen den Elektroden durchgeführt werden.

Ein Kontrollschaltkreis des implantierbaren Pulsgenerators erfasst solche elektrische Messungen, so dass das ärztliche Programmiergerät automatisch die einzelnen Leitungskörper identifizieren kann, die mit dem implantierbaren Pulsgenerator verbunden sind. Die elektrischen Messungen des Kontrollschaltkreises zur Identifikation der verbundenen Leitungskörper sind Feldpotentiale. Der Kontrollschaltkreis kann auch die Impedanz an jeder Elektrode messen, um die Kopplungseffizienz zwischen der jeweiligen Elektrode und dem Gewebe zu bestimmen und um die Fehlerkennung bezüglich der Verbindung zwischen der Elektrode und dem analogen Ausgabeschaltkreis des implantierbaren Pulsgenerators zu bestimmen. Bei dem bekannten System ist von Nachteil, dass die Identifikation nicht durch den implantierbaren Pulsgenerator selbst, sondern durch ein zusätzliches ärztliches Programmiergerät erfolgt.

Dokument US 2012/0123496 A1 beschäftigt sich mit der Erkennung der Verbindung und der Identifikation des Typs einer implantierten Leitung für eine implantierte medizinische Vorrichtung. Die Vorrichtung weist einen Prozessor auf, der die Verbindung sowie den Leitungstyp der Leitung bestimmen kann. Zunächst prüft ein Signalmessmodul die Verbindung der Leitungen, indem es Werte elektrischer Parameter während eines Signals zwischen zumindest zwei Elektroden prüft, insbesondere der Impedanz. Eine oder mehrere Leitungen können darin integrierte, aktive Elektronik aufweisen, die einen oder mehrere darin integrierte modulare Schaltkreise beinhaltet, je nachdem, ob die Leitung unipolar oder multipolar ist. Jeder der modularen Schaltkreise ist in der Lage, eine Vielzahl von Elektroden der Leitung zu steuern, und schließt eine Schaltungsanordnung ein, die elektrisch mit einer oder mehreren Elektroden der Leitung verbunden ist. Als solcher wirkt jeder der modularen Schaltkreise einer Leitung als Schnittstelle zwischen der implantierten medizinischen Vorrichtung und den Elektroden, mit denen der modulare Schaltkreis verbunden ist. Für die Messung der Impedanz steuert der Prozessor der Vorrichtung den modularen Schaltkreis, sodass dieser einen Spannungspuls zwischen einer ersten und einer zweiten Elektrode liefert. Das Signalmessmodul misst den resultierenden Strom und der Prozessor leitet den Impedanzwert ab. In einem weiteren Schritt sendet der Prozessor ein Abfragesignal entlang eines ersten Leiters der Leitung, um eine Antwort von den modularen Schaltkreisen über eine zweite Leitung zu erhalten. Eine solche Antwort von jedem modularen Schaltkreis liefert dem Prozessor Informationen zu dem modularen Schaltkreis und den Elektroden, die er steuert. In einem weiteren Konfigurationsschritt sendet der Prozessor ein Signal über die erste Leitung. Der Konfigurationsschritt schließt ein, dass die aktive Konfiguration der modularen Schaltkreise programmiert wird. Bezüglich Leitungsausführungen und darin verwendeter aktiver Elektronik bzw. modularer Schaltkreise wird auf das Dokument US 7,713,194 verwiesen. Gemäß dieser Druckschrift ist der modulare Schaltkreis derart ausgestaltet, dass er durch einen Bus gesteuert wird. Die Druckschrift US 2012/0123496 A1 beschreibt demnach, dass die zusätzliche Schnittstellenelektronik modularer Schaltkreise erkannt und somit die Elektrodenleitung bestimmt werden kann. Bei der bekannten Vorrichtung ist nachteilig, dass komplexe modulare Schaltkreise mit aktiver Elektronik zur Steuerung der Elektroden implementiert und programmiert werden müssen. Ferner beziehen sich die Informationen nur auf die modularen Schaltkreise und die damit verbundenen Elektroden, nicht aber auf die Leitung als Ganzes.

Ein Verfahren und eine Vorrichtung zur automatischen Erkennung von implantierbaren medizinischen Leitungen und deren Konfiguration sind in dem Dokument US 2003/0018369 A1 dargestellt. Dafür ist ein erster Kommunikationsschaltkreis, der Daten wie Modell- und Seriennummer, technische Information und Kalibrierungsdaten speichert, mit der Leitung verbunden oder in ihr integriert. Dieser weist einen Empfänger und einen Sender auf, um Datensignale von einer externen Quelle zu empfangen. So kann er bei der Herstellung mit Identifikationsdaten, Kalibrierungsdaten und anderen Daten programmiert werden. Der erste Kommunikationsschaltkreis ist als passiver Transponder ausgeführt und weist neben dem Receiver und Transmitter weiter einen Energiekoppler zur Energieversorgung und einen Steuerschaltkreis auf, welcher mit einem nicht-flüchtigen Speicher verbunden ist. Der Steuerschaltkreis liefert die in dem Speicher gespeicherten Informationen der Leitung zum Transmitter/Receiver des Transponders, der die Daten via RF oder anderer Kommunikation übermittelt. Während der Implantation der Leitung oder danach können die Informationen zu einem zweiten Kommunikationsschaltkreis außerhalb der Leitung transferiert werden. Die transferierten Daten können zur Identifikation der Leitung genutzt werden, in eine Patientenakte aufgenommen und in einen Zentralspeicher für die Nutzung durch Gesundheitsdienstleister transferiert werden. Anhand des Transponders kann die Leitung automatisch erkannt werden und die im Speicher abgelegten Daten können direkt transferiert und weitergegeben werden. Der Transponder benötigt neben einem Transmitter und Receiver allerdings eine separate Energieversorgung, eine Steuereinheit und einen programmierbaren, digitalen Speicher. Dadurch ist der Gesamtaufbau der Leitung vergleichsweise aufwendig und teuer.
Auch Dokument US 2014/0343633 A1 stellt eine elektrisch identifizierbare Elektrodenleitung mit einem Identifikationsmodul dar, welches zumindest einen Filter, einen Stromkonverter, eine Kommunikationsschaltung, einen Lastschalter und eine Speichereinheit wie ein EPROM zur Speicherung eines Identifikationscodes aufweist. Vor der Einbringung des Implantats wird jede Leitung implantiert und mit dem implantierbaren Pulsgenerator (oder einem externen Pulsgenerator) verbunden, welcher dann selbstidentifizierende Daten vom Identifikationsmodul ausliest und diese Information an eine externe Vorrichtung wie den Arztprogrammierer übermitteln kann. Dafür kann das Identifikationsmodul bis zu 32 Byte Daten speichern. Dieses Verfahren wird für jede Leitung, die implantiert wird, wiederholt. Das Identifikationsmodul nutzt zwei vorhandene Kontakte der Leitung zur Verbindung mit dem implantierbaren Pulsgenerator. Wie im zuvor genannten Dokument wird auch für diese bekannte Elektrodenleitung ein digitaler Speicher benötigt und der Aufbau des Identifikationsmoduls ist ähnlich komplex.

Aus den Dokumenten US 2006/0212096 A1, US 2008/0065181 A1 und US 7,983,763 B2 sind Vorrichtungen für die Identifizierung einer implantierbaren medizinischen Einrichtung und eines implantierten Leitersystems bekannt, bei dem ein RFID-Tag mit einem RFID-Chip in der Isolation, welche den Leiter umgibt, oder in dem Header einer implantierbaren Vorrichtung angeordnet ist. Ferner ist eine Leseeinrichtung vorgesehen, welche die in dem RFID-Chip gespeicherten Daten über das Gerät, das Leitersystem, den Hersteller oder den Patienten kabellos auslesen kann. Die auslesbaren Informationen enthalten jedoch keine Angaben über die aktuelle Anordnung und/oder den Anschluss der jeweiligen Elektroden. Zudem ist es bei den in diesen Dokumenten erläuterten Lösungen von Nachteil, dass vergleichsweise viel Energie für das Abfragen der Daten aus dem Implantat und für die Aktivierung des Chips aufgewendet werden muss. Hierfür werden zusätzliche Geräte eingesetzt, die eine erhebliche SAR-Belastung (SAR = specific absorption rate - Maß für die Absorption eines elektromagnetischen Feldes durch das Gewebe) des Patienten darstellen.

Dokument US 20070203547 A1 beschreibt eine Elektrodenleitung bei der ein Chip im Leitungskörper eingebettet ist.

Die Aufgabe der vorliegenden Erfindung besteht deshalb darin, eine Elektrodenleitung bzw. ein entsprechendes Implantat zu schaffen, so dass die Elektrodenleitung zuverlässig und eindeutig auch hinsichtlich ihrer Eigenschaften und der mit der Elektrodenleitung verbundenen Buchse bzw. dem mit der Elektrodenleitung verbundenen Kanal identifiziert werden kann. Das Implantat sowie die Elektrodenleitung sollen aber zugleich einfach aufgebaut sein, energiesparend arbeiten und kostengünstig hergestellt werden können. Die Elektrodenleitung soll möglichst wenig verändert werden. Die Aufgabe besteht ferner darin, ein einfaches Verfahren zur Identifikation einer Elektrodenleitung anzugeben, welches eine eindeutige Zuordnung einer Elektrodenleiter-Information zu einer Buchse des Implantats bzw. zu dem jeweiligen Kanal des Implantats (zum Beispiel Atrium, Ventrikel, Koronarsinus, Region des Rückenmarks oder des Gehirns, Magen, Blase, Vagusnerv, Phrenicusnerv oder Skeletmuskel) ermöglicht.

Die obige Aufgabe wird gelöst durch ein Implantat mit den Merkmalen des Anspruchs 1.

Die obige Aufgabe wird insbesondere gelöst durch ein Implantat, bei dem der Header im Bereich der mindestens einen Buchse mindestens ein elektromagnetisches Übertragungselement aufweist, das elektrisch mit einem Kontaktelement, welches an der Innenwand der mindestens einen Buchse vorgesehen ist verbunden ist, wobei das elektromagnetische Übertragungselement elektromagnetisch oder induktiv mit der Kommunikationsantenne gekoppelt ist.

Die elektromagnetische oder induktive Kopplung zwischen dem elektromagnetischen Übertragungselement und der Kommunikationsantenne wird dadurch begünstigt, dass der kleinste Abstand zwischen Kommunikationsantenne und elektromagnetischem Übertragungselement vorzugsweise kleiner als 12 mm ist. Es ist weiter von Vorteil, wenn die Kommunikationsantenne und das elektromagnetische Übertragungselement über einen Leiterabschnitt von mindestens 5 mm parallel verlaufen. Das elektromagnetische Übertragungselement ist vorzugsweise als Antenne oder elektrische Leiterkomponente (elektrischer Leiter, Leiterschleife) ausgebildet. Das elektromagnetische Übertragungselement und/oder die Kommunikationsantenne sind im oder am Header angeordnet, vorzugsweise in den Header eingebettet.

Um eine möglichst gute Kopplung zwischen der Kommunikationsantenne und dem elektromagnetischen Übertragungselement zu erzielen, wird das System in einem Ausführungsbeispiel so dimensioniert, dass an der Stelle der Übertragungsfrequenz die zwischen der Kommunikationsantenne und dem elektromagnetischen Übertragungselement übertragene Leistung maximal 10dB unterhalb des Maximums liegt, das sich bei Resonanz des Systems bestehend aus Kommunikationsantenne und elektromagnetischem Übertragungselement einstellen würde. Der magnetische Fluss soll in einem bevorzugten Ausführungsbeispiel die Kommunikationsantenne und das elektromagnetische Übertragungselement bestmöglich durchfluten, so dass der magnetische Kopplungsfaktor vorzugsweise mindestens 0,1 beträgt. Die Kommunikationsantenne kann beispielsweise auch für die Kommunikation zwischen Implantat und einem weiteren Gerät wie z. B. einem externen Kommunikationsgerät zur Kontaktierung eines Daten- und Servicecenters genutzt werden.

Das oben beschriebene erfindungsgemäße Implantat wird zur Erkennung der Elektrodenleitung verwendet, so dass kein zusätzliches Gerät hierfür erforderlich ist. Das erfindungsgemäße Implantat kommt zudem mit einer geringen Zahl zusätzlicher Bauelemente aus. Weiter wird der therapeutische Pfad durch die erfindungsgemäße Lösung nicht durch serielle galvanische Kopplung oder zusätzliche elektrische Komponenten gefährdet. Die erfindungsgemäße Lösung zeichnet sich zudem durch einen geringen Leistungsbedarf aus. Außerdem sind keine Vorkehrungen erforderlich, um die Funktion von EMI-Filtern gegen elektromagnetische Interferenz (EMI = elektromagnetische Interferenz) für das Auslesen der Information aus der Elektrodenleitung zu umgehen.

In einem Ausführungsbeispiel ist das elektromagnetische Übertragungselement über einen Kondensator an das Gehäuse des Implantats angeschlossen, das elektrisch leitfähig ausgebildet ist. Hierdurch werden hohe Frequenzen direkt auf das Gehäuse des Implantats abgeleitet. Dadurch können Kommunikationssignale nicht ins Gehäuse gelangen. Hierbei kann in einer ersten Alternative der Kondensator so dimensioniert sein, dass er bei der Übertragungsfrequenz, bei der das Übertragungselement mit der Kommunikationsantenne kommuniziert, kurzgeschlossen ist (d.h. der Betrag seiner Impedanz ist kleiner als 1,5 Ω). Bei dieser Alternative ist die Kapazität des Kondensators beispielsweise größer als 500 pF. In einer alternativen Ausführungsform wird der Kondensator so ausgelegt, dass eine bestmögliche Kopplung zwischen Kommunikationsantenne und elektromagnetischem Übertragungselement bei der Übertragungsfrequenz erreicht wird, d.h. die Übertragungsfrequenz liegt nahe der Resonanzfrequenz des Schaltkreises der Kommunikationsantenne. In diesem Fall ist die Kapazität des Kondensators vorzugsweise kleiner als 1,5 nF.

Um eine Kommunikation der Kommunikationsantenne mit einem externen Gerät (zum Beispiel für die Kontaktierung eines Daten- und Servicecenters oder Funktelemetrie) und eine Identifizierung einer angeschlossenen Elektrodenleitung zu ermöglichen, wobei die beiden Betriebsweisen vorzugsweise auch gleichzeitig erfolgen können, weist der Schaltkreis der Kommunikationsantenne vorzugsweise mindestens zwei Resonanzen auf. Hierdurch werden mindestens zwei getrennte Kanäle für die Kommunikationsantenne bereitgestellt.

Vorzugsweise weist das Implantat eine Vielzahl von Buchsen im Header auf und die Anzahl der elektromagnetischen Übertragungselemente korrespondiert mit der Anzahl der Buchsen, d.h. die Anzahl der Buchsen ist gleich der Anzahl der elektromagnetischen Übertragungselemente. Dabei ist vorzugsweise lediglich eine einzige Kommunikationsantenne im und/oder am Header vorgesehen, die mit allen im und/oder am Header angeordneten Übertragungselementen elektromagnetisch oder induktiv gekoppelt ist.

Wie unten im Detail beschrieben wird, erzeugt die Steuereinrichtung zur Identifikation der mindestens einen angeschlossenen Elektrodenleitung ein Abfragesignal und sendet dieses an die Kommunikationsantenne. In einem weiteren Ausführungsbeispiel ist die Steuereinrichtung derart eingerichtet, dass sie nach dem Auftreten eines vorbestimmten Ereignisses für jede Buchse der Vielzahl von Buchsen ein separates Abfragesignal erzeugt. Ein solches vorbestimmtes Ereignis ist beispielsweise das Anschließen der Elektrodenleitung, d.h. das vorschriftsmäßige Einstecken des Steckers in eine Buchse des Implantats bzw. die Herstellung einer galvanischen Verbindung zwischen einander entsprechenden Kontaktelementen des Steckers und der Buchse, in die der Stecker eingesteckt ist.

In einem weiteren Ausführungsbeispiel kann das Implantat eine Detektionseinheit aufweisen, welche das Anschließen der Elektrodenleitung an das Implantat detektiert. Die Detektionseinheit dient daher dazu, ein oben beschriebenes vorbestimmtes Ereignis zu erkennen. Vorzugsweise ist die Detektionseinheit mit der Steuereinrichtung des Implantats verbunden. Die Detektionseinheit kann beispielsweise ein Triggersignal an die Steuereinrichtung senden, wenn die Detektionseinheit erkennt, dass ein Stecker einer Elektrodenleitung vorschriftsmäßig in eine Buchse des Implantats eingesteckt wurde, sodass der galvanische Kontakt hergestellt ist.

Die obige Aufgabe wird ferner gelöst durch eine Elektrodenleitung mit einem Stecker zur Verbindung mit einem Implantat, mit mindestens einem elektrischen Leiter und einem den mindestens einen elektrischen Leiter isolierenden Isolationsschlauch, wobei die Elektrodenleitung außerdem einen
▪ in den Isolationsschlauch und/oder
▪ in den Stecker oder
▪ in einen isolierenden Körper eines mit dem Isolationsschlauch oder den Stecker vorzugsweise formschlüssig verbindbaren, separaten Zusatzteils eingebetteten,
hermetisch abgeschlossenen RFID-Chip aufweist, wobei der RFID-Chip elektrisch mit einer Leiterschleife oder mit mindestens einem an dem Stecker angeordneten Kontaktelement verbunden ist. Der RFID-Chip ist beispielsweise für UHF-Technologie, d.h. Frequenzen größer 800 MHz, geeignet. In der Schaltung des RFID-Chips ist keine RFID-Antenne vorgesehen. Erfindungsgemäß wird der RFID-Chip stattdessen beim Einstecken in eine Buchse des Implantats galvanisch oder über ein elektromagnetisches Feld und den elektrischen Leiter oder ein Kontaktelement der Elektrodenleitung mit dem elektromagnetischen Übertragungselement verbunden, welches im Header des Implantats angeordnet ist. Hierdurch wird der durch das zusätzliche Bauelement (RFID-Chip) benötigte Platz an der Elektrodenleitung minimiert. Die mechanische Belastung des RFID-Chips ist daher sehr gering. Das Zusatzteil mit dem RFID-Chip kann beispielsweise eine Länge von 500 µm und eine Wandstärke von 100 µm aufweisen. Vorzugsweise ist der RFID-Chip galvanisch mit dem Kontaktelement mit dem höchsten Induktivitäts- und/oder Widerstandsbelag verglichen mit anderen Kontaktelementen der Elektrodenleitung verbunden. Hierdurch werden die Verluste der mit dem RFID-Chip ausgetauschten Signale minimiert.

Der RFID-Chip der erfindungsgemäßen Elektrodenleitung weist vorzugsweise eine Speichereinheit zum Speichern von an das Implantat zu übertragenden Informationen auf. Alternativ können die zu übertragenden Informationen bei Aktivierung des RFID-Chips auch durch den Schaltkreis des RFID-Chips erzeugt werden.

Wie oben bereits erläutert wurde, ist in einem Ausführungsbeispiel der RFID-Chip mit einem Kontaktelement oder dem elektrischen Leiter der Elektrodenleitung galvanisch verbunden. Der elektrische Leiter der Elektrodenleitung besitzt ebenfalls eine galvanische Verbindung mit einem Kontaktelement. Daher ist das Kontaktelement vorzugsweise als ein therapeutisches Kontaktelement oder als ein von einem therapeutischen Kontaktelement verschiedenes, separates Kontaktelement ausgebildet. Jedes Kontaktelement, d.h. das Kontaktelement des RFID-Chips oder das Kontaktelement des elektrischen Leiters, ist beispielsweise als außen auf der Oberfläche des Steckers des Implantats liegende metallisierte Fläche, vorzugsweise aus Gold oder Platin, gestaltet. Jedes Kontaktelement bildet eine galvanische Verbindung mit einem entsprechenden Kontaktelement oder Pol, der an der Innenwand der Buchse des Implantats angeordnet ist, wenn der Stecker der Elektrodenleitung in die Buchse des Implantats vorschriftsmäßig eingesteckt ist. Das direkt mit dem RFID-Chip verbundene Kontaktelement kann beispielsweise als metallisierter Kontaktring ähnlich einer Kontakthülse für die Kontaktierung von Ringelektrode und Tipelektrode oder als eine metallisierte Dichtlippe oder mehrere metallisierte Dichtlippen ausgebildet sein.

In einer weiteren Ausführungsform kann der RFID-Chip zusätzlich galvanisch an ein größeres metallisches Element (zum Beispiel einen Metallring) angeschlossen sein, das kapazitiv mit einem elektrischen Leiter der Elektrodenleitung koppelt und somit als Groundplane des RFID-Chips dient. Das größere metallische Element hat eine Fläche von wenigstens 10 mm², wenn es sich bei dem größeren metallischen Element um eine flächige Ausführung handelt (z. B. ein Metallring). Bei z. B. meanderförmigen metallischen Elementen kann eine kleinere Oberfläche ausreichend sein. Diese kapazitive Kopplung erfolgt hierbei bevorzugt an eine andere elektrische Leitung als die, mit der der RFID-Chip galvanisch verbunden ist. Diese zweite elektrische Leitung ist vorzugsweise mit der Masse des Implantats verbunden, wenn die Elektrodenleitung in das Implantat eingesteckt ist. Beim Einstecken des Steckers der Elektrodenleitung in die Buchse des Implantats wird über das metallische Element und die kapazitiv Kopplung somit eine Verbindung des RFID-Chips mit der Masse des Implantats erreicht.

Wenn in einer alternativen Ausführungsform der RFID-Chip mit einer Leiterschleife verbunden ist, dann ist diese derart gestaltet, dass eine elektromagnetische Kopplung mit einem elektrischen Leiter der Elektrodenleitung besteht.

Durch das vorschriftsmäßige Einstecken des Steckers der Elektrodenleitung in die Buchse des Implantats ist erfindungsgemäß eine räumlich enge Anordnung zwischen RFID-Chip, Kommunikationsantenne und Übertragungselement gegeben, sodass auch bei schlechter Kontaktierung eine gute Kopplung der genannten Elemente besteht.

In einer bevorzugten Ausführungsform ist der RFID-Chip auf der einen Seite mit einem ersten Kontaktelement und auf der anderen Seite mit einem zweiten Kontaktelement der Elektrodenleitung verbunden. Entsprechend weist das Implantat ein erstes elektromagnetisches Übertragungselement, das elektrisch mit dem ersten Kontaktelement (Pol, Anschluss) der Buchse verbunden ist, und ein zweites elektromagnetisches Übertragungselement auf, das elektrisch mit dem zweiten Kontaktelement der Buchse verbunden ist. Beide elektromagnetische Übertragungselemente können für die Identifikation einer Elektrodenleitung verwendet werden.

Es ist von Vorteil, wenn der RFID-Chip aktiv oder passiv ausgebildet ist. Unter einem aktiven RFID-Chip wird ein RFID-Chip verstanden, der nach Erhalt eines Signals über das elektromagnetische Übertragungselement, das galvanisch oder elektromagnetisch mit dem RFID-Chip verbunden ist, direkt (d.h. unmittelbar, ohne ein zweites Signal abzuwarten) ein Antwortsignal erzeugt. Demgegenüber wird ein passiver RFID-Chip durch ein Triggersignal des elektromagnetischen Übertragungselements zunächst lediglich freigeschaltet. Der passive RFID-Chip ist erst nach einem derartigen Freischalten (Entsperren) in der Lage, aus einem (weiteren) Signal des elektromagnetischen Übertragungselements Energie zu entnehmen und ein entsprechendes Antwortsignal zu erzeugen.

In dem Fall, dass es sich bei dem Implantat um einen Herzschrittmacher (Pacemaker) handelt, kann für die Energieübertragung, als Abfragesignal oder als Triggersignal für einen passiven RFID-Chip ein Pace-Signal verwendet werden. Der Verbrauch des RFID-Chips ist dabei vorzugsweise so gestaltet, dass für die Erzeugung eines Antwortsignals weniger als 1 % der Energie des Pace-Signals erforderlich ist. In seiner Funktion als Triggersignal kann das Pace-Signal durch den RFID-Chip hinsichtlich seiner Amplitude, seines Pulsabstands und/oder seiner Pulsbreite untersucht und abhängig von dem jeweils ermittelten Amplitudenwert, Pulsabstandswert oder Pulsbreitenwert eine Freischaltung des RFID-Chips erfolgen. Die Bestimmung des Amplitudenwerts eines Pace-Signals kann beispielsweise mittels einer im RFID-Chip angeordneten Kapazitätsdiode realisiert werden, welche nur beim Anliegen einer Spannung in einem vorgegebenen Wertebereich die richtige Abstimmung des RFID-Chips Sendekreises auf die festgelegte Kommunikationsfrequenz realisiert. In Bezug auf die Pulsabstandsmessung wird nur dann die Erzeugung eines Antwortsignals durch den RFID-Chip zugelassen (nach Anregung über die Kommunikationsantenne und das elektromagnetische Übertragungselement), wenn der Abstand zweier Pulse eines Pace-Signals oder mehrerer Pace-Signale in einem vorgegebenen Wertebereich liegt. Gleiches gilt analog für die Pulsbreite. Hierbei ist bevorzugt, wenn zwei aufeinanderfolgende Pace-Signale durch das Implantat derart erzeugt werden, dass ihr Abstand kürzer ist als die absolute Refraktärzeit des Muskelgewebes. Diese Signale haben physiologisch keine Auswirkung. Es ist weiter bevorzugt, dass dann, wenn der Herzschrittmacher mit LV Pacing VV Delay Null betrieben wird, für ein Triggersignal einmalig ein VV Delay verschieden von Null verwendet wird.

Die obige Aufgabe wird außerdem durch ein Verfahren zur Identifikation einer oben beschriebenen Elektrodenleitung mittels eines oben beschriebenen Implantats gelöst, wobei das Implantat mit der Elektrodenleitung verbunden ist, umfassend die folgenden Schritte:
- Erzeugen eines elektromagnetischen Abfragesignals durch die Steuereinrichtung und Weiterleiten an die mit der Steuereinrichtung verbundene Kommunikationsantenne,
- Senden des elektromagnetischen Abfragesignals durch die Kommunikationsantenne, beispielsweise eines hochfrequenten Signals im geeigneten Frequenzbereich (zum Beispiel bei 860 MHz),
- Empfangen des Abfragesignals durch das Übertragungselement mittels elektromagnetischer oder induktiver Kopplung,
- Weiterleiten des empfangenen Abfragesignals an den RFID-Chip,
- Verarbeiten des empfangenen Abfragesignals durch den RFID-Chip,
- Erzeugen eines entsprechenden elektromagnetischen Antwortsignals durch den RFID-Chip und Übertragen des Antwortsignals an das Übertragungselement,
- Empfangen des Antwortsignals durch die Kommunikationsantenne des Implantats mittels elektromagnetischer oder induktiver Kopplung mit dem Übertragungselement und Weiterleiten an die Steuereinrichtung sowie
- Verarbeiten des weitergeleiteten empfangenen Antwortsignals in der Steuereinrichtung.

Das erfindungsgemäße Verfahren ist ein einfaches Verfahren, mit dem es möglich ist, dass Informationen zu einer Elektrodenleitung mittels des Implantats identifiziert werden können. Durch das erfindungsgemäße Verfahren wird kein zusätzliches Gerät zum Abfragen der Elektrodenleitungen benötigt. Die Abfrage erfolgt durch das Implantat selbst und ermöglicht eine Zuordnung der Elektrodenleitung zu einem Kanal des Implantats. Das Konzept kommt zudem mit wenig Energie aus, sodass die durch den Energiespeicher des Implantats bereitgestellte Energie für die Sensorik und die Therapie genutzt werden kann.

Weiter wird die SAR-Belastung (SAR = spezifische Absorptionsrate) des Patienten minimiert.

In einem Ausführungsbeispiel des erfindungsgemäßen Verfahrens erfolgt die Erzeugung des Abfragesignals durch die Steuereinrichtung nach dem Auftreten eines vorbestimmten Ereignisses, vorzugsweise nach dem Anschließen einer Elektrodenleitung an das Implantat, d. h. wenn ein (dauerhafter) galvanischer Kontakt zwischen dem mindestens einen elektrischen Leiter einer Elektrodenleitung und einem zugehörigen Kontaktelement oder Pol, der an der Innenwand einer Buchse des Implantats angeordnet ist, hergestellt ist.

Die Herstellung eines solchen galvanischen Kontakts kann mittels einem der nachfolgenden Verfahren oder einer Kombination der nachfolgend beschriebenen Verfahren detektiert werden. Diese Erkennung erfolgt vorzugsweise mittels der oben beschriebenen Detektionseinheit. Diese ist vorzugsweise mit der Steuereinrichtung verbunden oder in diese integriert und übermittelt das Ergebnis des Detektionsverfahrens an die Steuereinrichtung.
i) Die Detektionseinheit misst eine Elektrodenimpedanz (bei einer Frequenz kleiner als 1 MHz) zwischen zwei an einer Buchse angeordneten Kontaktelementen. Wenn die Elektrodenimpedanz im Bereich zwischen 10 Ω und 2000 Ω liegt, stellt die Detektionseinheit einen galvanischen Kontakt fest.
ii) Die Detektionseinheit misst Spannungssignale zwischen zwei an einer Buchse angeordneten Kontaktelementen. Wenn die gemessene Spannung zwischen 0,5 mV und 200 mV beträgt, stellt die Detektionseinheit einen galvanischen Kontakt fest.
iii) Die Detektionseinheit misst die Periodizität der von der Detektionseinheit angelegten Spannungssignale zwischen zwei an einer Buchse angeordneten Kontaktelementen. Wenn die Periodizität im Wertebereich zwischen 20 bpm und 200 bpm liegt, stellt die Detektionseinheit einen galvanischen Kontakt fest.
iv) Die Detektionseinheit misst kontinuierlich oder in vorgegebenen, festen Abständen die Resonanzfrequenz der Kommunikationsantenne. Falls die Detektionseinheit eine Verschiebung (Verstimmung) der Resonanzfrequenz der Antenne um mehr als 50% des sonst gemessenen Wertes feststellt, wurde ein galvanischer Kontakt hergestellt.

Die in den obigen Möglichkeiten zur Feststellung eines galvanischen Kontakts zwischen dem elektrischen Leiter einer Elektrodenleitung und einem Kontaktelement oder Pol einer Buchse des Implantats angegebenen Werte sind beispielhaft zu verstehen. Die Wertebereiche für die Erkennung des Kontakts können für das jeweilige Implantat oder den jeweiligen Implantattyp vorab individuell festgelegt und/oder von außen einprogrammiert (neu eingegeben oder geändert) werden.

In einem weiteren Ausführungsbeispiel des erfindungsgemäßen Verfahrens ist vorgesehen, dass dann, wenn zwei oder mehr als zwei Elektrodenleitungen an das Implantat angeschlossen sind, wobei von diesen Elektrodenleitungen eine Elektrodenleitung zuletzt an das Implantat angeschlossen ist, das oben beschriebene Verfahren zur Identifikation für alle angeschlossenen Elektrodenleitungen nacheinander durchgeführt wird und dass das jeweilige Antwortsignal mit den bereits in einer Speichereinrichtung des Implantats gespeicherten Informationen zu den Elektrodenleitungen verglichen und basierend auf dem Ergebnis des Vergleichs eine Zuordnung der Informationen der zuletzt angeschlossenen Elektrodenleitung zu der jeweiligen Buchse des Implantats erfolgt. Durch eine interne Logik kann zudem eine Zuordnung der Kanäle des Implantats zu den eingesteckten Elektrodenleitungen vorgenommen werden. Dies kann beispielsweise dadurch realisiert werden, dass ein Vergleich des Ergebnisses des letzten getriggerten Lesevorgangs mit vorangegangenen Lesevorgängen die Identifizierung einer zuletzt angeschlossenen Elektrodenleitung erlaubt. Zusammen mit einer in einem Speicher des Implantats gespeicherten Information darüber, welcher Kanal zuletzt angeschlossen wurde, ist eine Verknüpfung der Elektrodeninformation mit dem Kanal möglich.

In einem weiteren Ausführungsbeispiel des erfindungsgemäßen Verfahrens wird die Abfrage der Elektrodenleitung durch Erzeugung des elektromagnetischen Abfragesignals in einem begrenzten Zeitfenster nach dem vorbestimmten Ereignis, beispielsweise über einen Zeitraum von 1 Sekunde, durchgeführt, um die Energiereserven des Implantats nicht unnötig durch die Elektrodenleitung-Abfrage zu belasten. Alternativ oder zusätzlich kann die Abfrage der Elektrodenleitung abhängig von dem Signal eines im Gehäuse des Implantats angeordneten Bewegungssensors gesteuert werden. Erkennt der Bewegungssensor beispielsweise bei einer Bewegung die kleiner ist als ein vorgegebener Schwellwert eine Ruhephase des Implantats, so wird die Abfrage der Elektrodenleitung unterbrochen. Hierbei geht man davon aus, dass das Einstecken eines Steckers einer Elektrodenleitung in das Implantat mit einer Bewegung des Implantats verbunden ist.

Alternativ oder zusätzlich zu dem oben erläuterten Vorgehen kann eine Zuordnung der Buchse des Implantats zu einer Elektrodenleitung und ihrer zugehörigen Information dadurch erfolgen, dass die Empfangsstärke des Antwortsignals, das mittels der Kommunikationsantenne empfangen und an die Steuereinrichtung weitergeleitet wird, herangezogen wird.

In einem weiteren Ausführungsbeispiel kann das erfindungsgemäße Verfahren und das erfindungsgemäße Implantat dazu verwendet werden, einen Blindstecker zu identifizieren. Ein Blindstecker kann eingesetzt werden, wenn nicht alle Buchsen eines Implantats mit einem Stecker für eine Elektrodenleitung genutzt werden. In so einem Fall wird eine nicht genutzte Buchse des Implantats mit einem Blindstecker belegt. Dies dient dem Zweck eine nicht genutzte Buchse zu verschließen, so dass keine Flüssigkeit in die Buchse eindringt. Analog wie bei einer Elektrodenleitung kann auch ein Blindstecker mit einem RFID-Chip nach obigem Beispiel ausgestattet werden. Ein derartig ausgestatteter Blindstecker ist dann wie eine Elektrodenleitung identifizierbar. Damit kann sowohl eine Zuordnung des Blindsteckers zu einer bestimmten Buchse des Implantats stattfinden als auch der Blindstecker als solches erkannt werden.

Durch die erfindungsgemäße Elektrodenleitung, das erfindungsgemäße Implantat sowie das erfindungsgemäße Verfahren können auf einfache und kostengünstige Weise Informationen die Elektrodenleitung betreffend ausgelesen werden. Durch den Signalweg über ein Übertragungselement und eine Kommunikationsantenne ist der Leistungsbedarf zum Auslesen der Informationen deutlich reduziert. Hierdurch wird auch die SAR-Belastung des Patienten verringert. Weiter ist es bei der erfindungsgemäßen Lösung nicht erforderlich, in das Implantat integrierte EMI-Schutzkondensatoren während einer solchen Abfrage von Daten der angeschlossenen Elektrodenleitung abzuschalten.

Es zeigen schematisch:
- Fig. 1: ein erstes Ausführungsbeispiel einer erfindungsgemäßen Elektrodenleitung und eines erfindungsgemäßen Implantats in einer perspektivischen Ansicht von der Seite bzw. in einer Schnittdarstellung,
- Fig. 2: zweites bis viertes Ausführungsbeispiel einer erfindungsgemäßen Elektrodenleitung, jeweils in einer perspektivischen Ansicht von der Seite,
- Fig. 3: ein fünftes Ausführungsbeispiel einer erfindungsgemäßen Elektrodenleitung sowie ein zweites Ausführungsbeispiel eines erfindungsgemäßen Implantats in einer perspektivischen Ansicht von der Seite bzw. in einer Schnittdarstellung,
- Fig. 4: ein sechstes Ausführungsbeispiel einer erfindungsgemäßen Elektrodenleitung sowie ein drittes Ausführungsbeispiel eines erfindungsgemäßen Implantats in einer perspektivischen Ansicht von der Seite bzw. in einer Schnittdarstellung,
- Fig. 5: ein siebtes Ausführungsbeispiel einer erfindungsgemäßen Elektrodenleitung sowie ein viertes Ausführungsbeispiel eines erfindungsgemäßen Implantats in einer perspektivischen Ansicht von der Seite bzw. in einer Schnittdarstellung,
- Fig. 6: ein achtes Ausführungsbeispiel einer erfindungsgemäßen Elektrodenleitung sowie ein fünftes Ausführungsbeispiel eines erfindungsgemäßen Implantats in einer perspektivischen Ansicht von der Seite bzw. in einer Schnittdarstellung,
- Fig. 7: ein neuntes Ausführungsbeispiel einer erfindungsgemäßen Elektrodenleitung sowie ein sechstes Ausführungsbeispiel eines erfindungsgemäßen Implantats in einer perspektivischen Ansicht von der Seite bzw. in einer Schnittdarstellung,
- Fig. 8: ein zehntes Ausführungsbeispiel einer erfindungsgemäßen Elektrodenleitung sowie ein siebtes Ausführungsbeispiel eines erfindungsgemäßen Implantats in einer perspektivischen Ansicht von der Seite bzw. in einer Schnittdarstellung,
- Fig. 9: ein elftes Ausführungsbeispiel einer erfindungsgemäßen Elektrodenleitung sowie ein achtes Ausführungsbeispiel eines erfindungsgemäßen Implantats in einer perspektivischen Ansicht von der Seite bzw. in einer Schnittdarstellung und
- Fig. 10: ein zwölftes Ausführungsbeispiel einer erfindungsgemäßen Elektrodenleitung sowie ein neuntes Ausführungsbeispiel eines erfindungsgemäßen Implantat in einer perspektivischen Ansicht von der Seite bzw. in einer Schnittdarstellung.

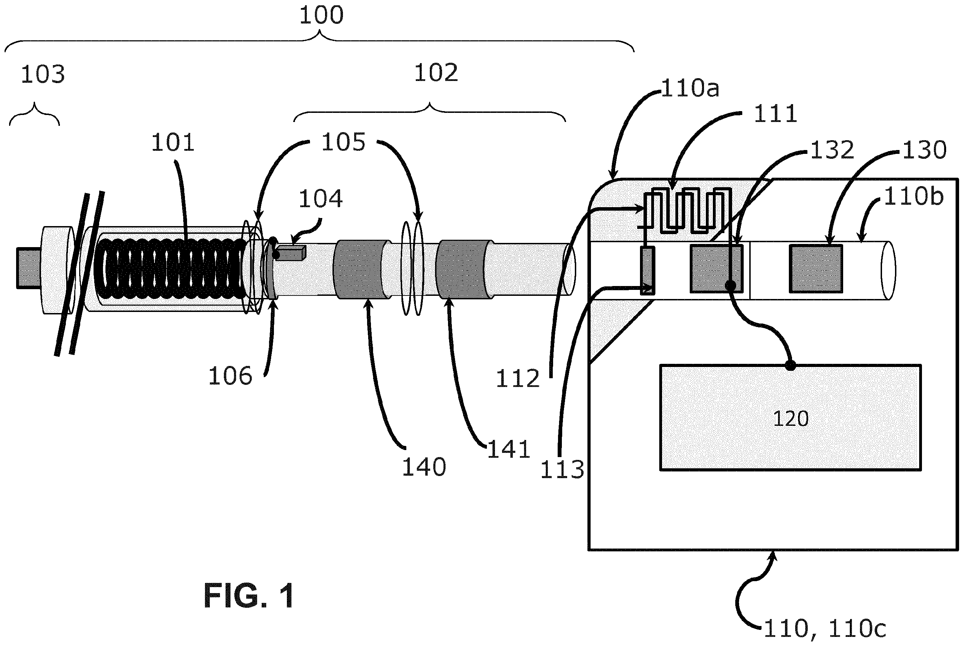

Fig. 1 zeigt ein erstes Ausführungsbeispiel einer erfindungsgemäßen Elektrodenleitung 100 (z. B. Elektrodenleitung zur Brady- oder Tachykardiebehandlung oder Neurostimulation) mit einem elektrischen Leiter 101. Alternativ können auch mehrere elektrische Leiter 101 vorgesehen sein. Am distalen Ende 103 der Elektrodenleitung 100 ist eine Elektrodenspitze angeordnet, welche den elektrischen Kontakt zur Umgebung, beispielsweise dem Gewebe des Patienten, herstellt. Die Elektrodenspitze kann als Stimulations-, Mess- oder Ableitelektrode ausgebildet sein. Am proximalen Ende der Elektrodenleitung 100 ist ein Stecker 102 angeordnet, welcher in eine Buchse 110b eines ebenfalls gezeigten aktiven Implantats 110 eingesteckt werden kann. Die Buchse 110b ragt in einen Header 110a des Implantats 110 hinein. Häufig weist das Implantat 110 mehrere Buchsen 110b auf, die jeweils mit einer Elektrodenleitung 100 verbunden werden können. Das aktive Implantat 110 kann beispielsweise als Herzschrittmacher oder Defibrillator ausgebildet sein. Durch den Stecker 102 besteht eine mechanische und elektrische Verbindung zwischen der Elektrodenleitung 100 und dem aktiven Implantat 110. Über entsprechende Kontaktelemente 140, 141 (Pole, Anschlüsse) des Steckers 102 und Kontaktelemente 130, 131 (Pole, Anschlüsse) der Buchse besteht eine elektrisch leitende (galvanische) Verbindung zwischen dem elektrischen Leiter 101 der Elektrodenleitung 100 und den innenliegenden elektrischen Komponenten des Implantats 110, beispielsweise einer Steuereinrichtung 120.

In den Stecker 102 (zum Beispiel IS1-, IS4/DF4-Stecker) ist ein RFID-Chip 104 integriert. Dies hat den Vorteil, dass der RFID-Chip 104 nach dem Einstecken des Steckers 102 in die Buchse 110b des Implantats 110 innerhalb des steifen Headers 110a des Implantats 110 angeordnet ist, in dem die Buchse 110b vorgesehen ist, und somit vor hoher mechanischer Belastung (Biegung/Abrieb) geschützt ist. Der RFID-Chip 104 ist bis auf ein mit dem RFID-Chip elektrisch leitend verbundenes Chip-Kontaktelement 106 hermetisch abgeschlossen von der Isolierung des Steckers 102 umhüllt. Das Chip-Kontaktelement 106 besteht beispielsweise aus einer außen am Stecker 102 der Elektrodenleitung 100 angeordneten Metallisierung in Form eines Rings, enthaltend vorzugsweise Gold und/oder Platin. Das Chip-Kontaktelement 106 ist in einem Abstand von den therapeutisch genutzten Kontaktelementen 140, 141 des Steckers 102 entfernt angeordnet.

Alternativ kann der RFID-Chip 104 zwischen einem beispielsweise als Silikonschlauch ausgeführten Isolationsschlauch und einer am proximalen Ende der Elektrodenleitung 100 im Bereich des Steckers 102 vorgesehenen, nicht dargestellten Isolationshülse angeordnet sein. Die Isolationshülse kann als separates Zusatzteil ausgeführt sein, welches über den Isolationsschlauch schiebbar ist. Das Zusatzteil kann beispielsweise eine Länge von 500 µm und eine Wandstärke von 100 µm aufweisen. Die Isolationshülse, welche beispielsweise aus einem Flüssigkristallpolymer (LCP), Silikon, einer Keramik und/oder Glas besteht, umgibt derart den Isolationsschlauch, dass der RFID-Chip 104 hermetisch gegenüber der Umgebung abgeschlossen ist.

Als RFID-Chip 104 kann zum Beispiel ein Chip für den Frequenzbereich zwischen 840 MHz und 960 MHz verwendet werden. Weiter kann eine Speicherkapazität von 512 Bit frei beschreibbar und 240 Bit zur Hinterlegung des Electronic Product Code (EPC) vorgesehen sein. Der Speicher des RFID-Chips 104 kann sowohl bei der Herstellung im Werk als auch während der Ausführung des erfindungsgemäßen Verfahrens zur Identifikation einer Elektrodenleitung gelesen und/oder beschrieben werden. Im Speicher des RFID-Chips 104 können Informationen zur Identifikation der Elektrodenleitung 100 sowie zu Ihrer Verwendung enthalten sein. Derartige Informationen können umfassen: den Hersteller, den Typ der Elektrodenleitung, die Seriennummer, das Herstellungsdatum, Zulassungsregionen, Zulassungsbedingungen, Implantationsdatum, Implantationskompatibilitäten, MRI-Kompatibilität und dergleichen. Weiter kann der Speicher des RFID-Chips 104 Sicherheitsmechanismen und Sicherheitsinformationen enthalten, die die Integrität (zum Beispiel bei partiellem Datenverlust) und die Echtheit (zum Beispiel bei Manipulation) der gespeicherten Informationen sicherstellt beziehungsweise erkennbar macht.

Das Implantat 110 enthält, wie oben bereits erwähnt, in einem hermetisch nach außen abgeschlossenen Gehäuse 110c eine Steuereinrichtung 120, welche mit dem Kontaktelement 132 oder den Kontaktelementen 130, 132 für die Elektrodenleitung 100 verbunden ist. Nach Einstecken einer Elektrodenleitung 100 in eine entsprechende Buchse 110b in dem Headerbereich 110a des Implantats 110 besteht eine elektrisch leitende (galvanische) Verbindung zwischen der Steuereinrichtung 120 und dem einen elektrischen Leiter 101 oder den mehreren elektrischen Leitern 101 der Elektrodenleitung 100. Diese erfolgt über Durchkontaktierungen in das Innere des gekapselten Implantats 110.

Im Header 110a des Implantats 110 ist ferner eine Kommunikationsantenne 111 vorgesehen, die auf die Kommunikationsfrequenz abgestimmt ist. Die Kommunikationsantenne 111 kann auch als Antenne zur Datenübertragung an einen externen Empfänger für die Kontaktierung eines Daten- und Servicecenters verwendet werden. Die Kommunikationsantenne 111 ist über eine Durchkontaktierung (Feedthrough) elektrisch leitend mit der Steuereinrichtung 120 verbunden. In einer bevorzugten Ausführungsform ist der Schaltkreis der Kommunikationsantenne 111 so gestaltet, dass er mindestens zwei Resonanzen aufweist, sodass zwei getrennte Kanäle für die Kommunikation mit den Elektrodenleitungen 100 und für die Kommunikation zu anderen Zwecken wie die Kommunikation mit einem externen Gerät zur Kontaktierung eines Daten- und Servicecenters oder für die Funktelemetrie zur Verfügung stehen, insbesondere auch gleichzeitig.

Zusätzlich ist bei dem in Fig. 1 dargestellten Ausführungsbeispiel im Header 110a ein elektromagnetisches Übertragungselement in Form einer zweiten Antenne 112 vorgesehen. Die Antenne 112 ist derart angeordnet, dass sie möglichst optimal bei der verwendeten UHF RFID-Frequenz mit der Kommunikationsantenne 111 elektromagnetisch oder induktiv koppelt. Die Antenne 112 ist galvanisch mit einem an der inneren Oberfläche der Buchse 110b angeordneten Kontaktelement (Pol, Anschluss) 113 verbunden, welches beabstandet von den therapeutischen Kontaktelementen 130, 132 angeordnet ist.

Nach dem korrekten Einstecken des Steckers 102 in die Buchse 110b wird das Chip-Kontaktelement 106, das mit dem RFID-Chip 104 verbunden ist, mit dem Kontaktelement 113 der Buchse 110b galvanisch verbunden. Hierdurch entsteht eine galvanische Verbindung zwischen dem RFID-Chip 104 und der Antenne 112.

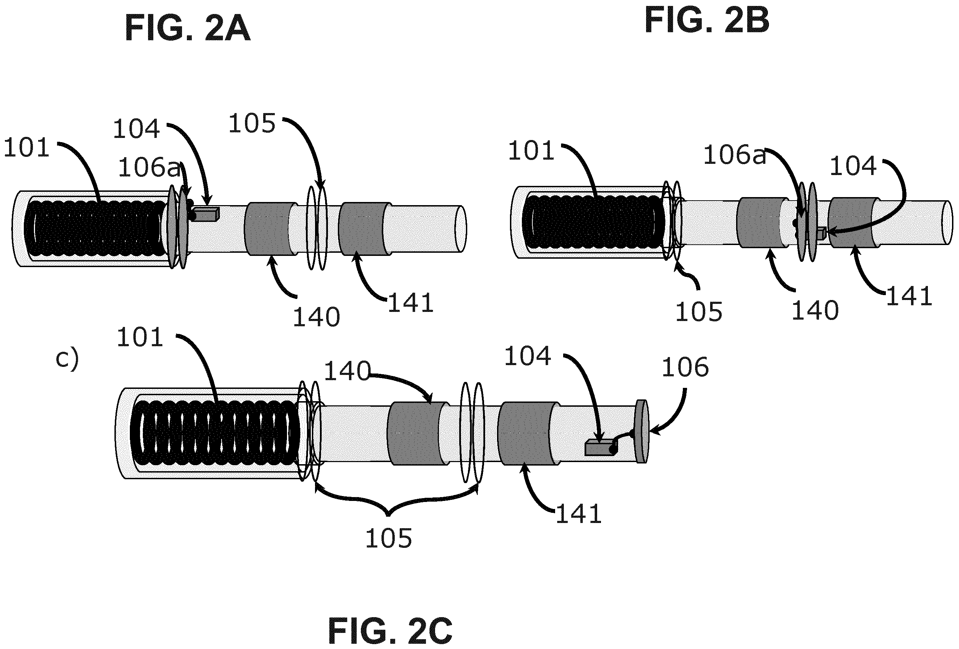

Der Kontakt zwischen dem RFID-Chip 104 und der im Header 110a angeordneten Antenne 112 kann auch auf andere Weise beim Einstecken des Steckers 102 in die Buchse 110 b realisiert werden. Eine alternative Möglichkeit ist beispielsweise anhand von Fig. 2c) dargestellt. Das ringförmige Chip-Kontaktelement 106, das mit dem RFID-Chip 104 verbunden ist, ist in diesem Ausführungsbeispiel am proximalen Ende des Steckers 102 angeordnet. Alternativ kann die Kontaktierung, wie in den Figuren 2a) und b) dargestellt, über metallisierte Dichtlippen 106a, die ebenfalls galvanisch mit dem RFID-Chip 104 verbunden sind, realisiert werden. Darüber hinaus können weitere Dichtlippen 105 zur Abdichtung der Steckverbindung vorgesehen sein.

Das aktive Implantat 110 besitzt ferner eine nicht dargestellte Filtereinheit gegen elektromagnetische Interferenz (EMI-Filter), um beispielsweise das Eindringen von Mobiltelefonsignalen in das Innere des gekapselten Gehäuses des Implantats 110 zu verhindern. Die Filtereinheit schließt hochfrequente Signale (Frequenz > 1 kHz) auf die elektrische Masse kurz. Hierfür werden zum Beispiel parallel geschaltete Kondensatoren mit hoher Kapazität eingesetzt.

Im Folgenden soll das erfindungsgemäße Verfahren zur Identifikation einer Elektrodenleitung mittels des in Fig. 1 gezeigten Implantat 110 erläutert werden.

Wenn die Steuereinrichtung 120 des Implantats mittels eines unten beschriebenen Verfahrens erkennt, ob zuvor eine Elektrodenleitung 100 an eine Buchse 110b des Implantats 110 angeschlossen wurde, wird das erfindungsgemäße Identifikationsverfahren für die Elektrodenleitung gestartet. Die Steuereinrichtung 120 erzeugt ein elektromagnetisches Abfragesignal, das an die Kommunikationsantenne 111 weitergeleitet und durch diese gesendet wird. Bei einem derartigen Abfragesignal kann es sich beispielsweise um ein hochfrequentes Signal in einem geeigneten Frequenzbereich (zum Beispiel 860 MHz) handeln. Das Abfragesignal koppelt durch die lokale Nähe in die Antenne 112 und wird von dieser über die galvanische Verbindung zu dem RFID-Chip 104 der eingesteckten Elektrodenleitung 100 weitergeleitet. Der RFID-Chip 104 wird durch das Abfragesignal aktiviert und verarbeitet das Abfragesignal. Das aufgrund dieser Aktivierung durch den RFID-Chip 104 generierte Antwortsignal enthaltend beispielsweise die oben angegebenen Informationen zur Elektrodenleitung 100, die dem Speicher des RFID-Chips 104 entnommen werden, wird durch den RFID-Chip 104 an die Antenne 112 im Header 110a des Implantats 110 übertragen und von dieser gesendet. Durch die elektromagnetische Kopplung zwischen Antenne 112 und Kommunikationsantenne 111 im Header 110a empfängt die Kommunikationsantenne 111 das von der Antenne 112 ausgesandte Antwortsignal und überträgt dieses an die Steuereinrichtung 120. Die Steuereinrichtung 120 wertet das übertragene Antwortsignal aus und ordnet die von der Elektrodenleitung 100 erhaltenen Informationen zur Identifikation der Elektrodenleitung 100 der jeweiligen Buchse 110b bzw. dem Kanal zu.

Durch die räumlich nahe Anordnung zwischen hermetisch gekapseltem RFID-Chip 104, Antenne 112 und Kommunikationsantenne 111 ist auch bei schlechter galvanischer Kontaktierung der Kontaktelemente von Stecker 102 und Buchse 110b eine ausreichend gute Kopplung realisierbar. Typischerweise beträgt der kleinste Abstand zwischen der Antenne 112 und der Kommunikationsantenne 111 weniger als 12 mm.

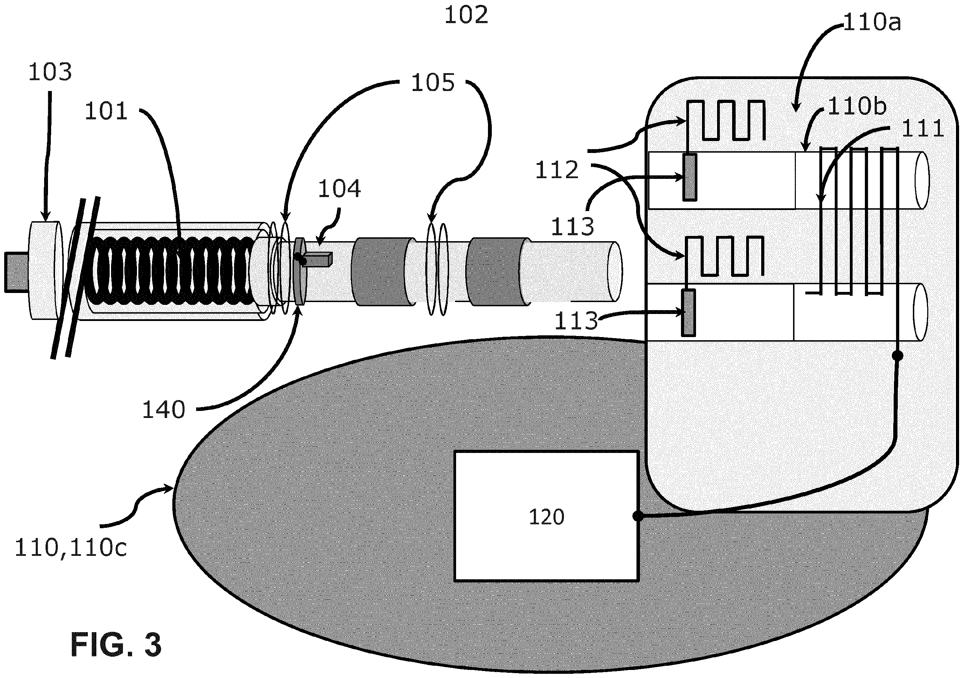

Sind in dem Header 110a des Implantats mehrere Buchsen 110b angeordnet (siehe Fig. 3), so erfolgt die Zuordnung der einzelnen Elektrodenleitungen 100 zu den Buchsen 110b des Implantats 110 bzw. dem jeweiligen Kanal über eine Logik innerhalb des Implantats 110. Die neu angeschlossene Elektrodenleitung 100 wird mit Hilfe eines Vergleichs mit dem letzten getriggerten Identifikationsvorgang identifiziert und einer Abfrage aller eingesteckten Elektrodenleitungen nacheinander. Für eine derartige Abfrage weist der Header 110 a des in Fig. 3 dargestellten Implantats 110 zu jeder Buchse 110b eine separate Antenne 112 auf, um die räumliche Zuordnung der übertragenen Signale zu einer bestimmten Buchse zu ermöglichen. Demgegenüber ist lediglich eine einzige Kommunikationsantenne 111 vorgesehen, welche, wie in Fig. 3 gezeigt, sich derart über den Header 110a erstreckt, dass sie mit beiden Antennen 112 elektromagnetisch oder induktiv gekoppelt ist. Die Steuereinrichtung 120 des Implantats vergleicht die in einem Speicher gespeicherten Informationen zu den Elektrodenleitungen 100 aus vorherigen Abfrageverfahren mit den empfangenen Antwortsignalen der Elektrodenleitungen 100 und ordnet auf der Basis des Ergebnisses dieses Vergleichs die Informationen zur neu angeschlossenen Elektrodenleitung 100 den entsprechenden Kontaktelementen des Implantats 110 der jeweiligen Buchse 110b zu. Hinsichtlich der Zuordnung zu dem Kanal des Implantats 110, mit dem die jeweilige Elektrodenleitung kontaktiert wurde, ist es erforderlich, dass die Steuereinrichtung 120 des Implantats 110 eigenständig erkennt, ob und an welchen Kanal eine Elektrodenleitung angeschlossen wird. Basierend auf den Informationen zu der neu angeschlossenen Elektrodenleitung sowie der Kenntnis darüber, welcher Kanal als letztes angeschlossen wurde, ist eine Verknüpfung der Informationen zu der Elektrodenleitung mit dem angeschlossenen Kanal möglich.

Um sicherzustellen, dass die Elektrodenleitung 100 in einer ausreichenden Zeitauflösung detektiert wird, ohne die Energiereserven des Implantats 110 unnötig durch eine zu hochfrequente Abfrage zu belasten, kann der Implantationsvorgang, der mit einem Einstecken der Elektrodenleitungen verbunden ist, über einen im Implantat 110 angeordneten Lagesensor und/oder einen Bewegungssensor im Implantat 110 detektiert werden. Entsprechend wird mittels der Steuereinrichtung 120 nur dann ein Abfragesignal erzeugt, wenn der Lagesensor und/oder der Bewegungssensor ermitteln, dass eine Implantation durchgeführt wird. Werden aufgrund des Vorhandenseins mehrerer Buchsen 110b mehrere Abfragesignale erzeugt, so beträgt der zeitliche Abstand der Abfragen vorzugsweise weniger als 1 Sekunde. Nach einer erfolgreichen Identifikation der Elektrodenleitung oder der Elektrodenleitungen wird das hochfrequente Abfragen der betroffenen Kanäle abgeschaltet. In einem weiteren Ausführungsbeispiel kann eine vorgebbare Ruhephase nach einer abgeschlossenen Abfrage in zeitlicher Hinsicht festgelegt werden, bei der die Bewegung geringer ist als ein vorgebbarer Schwellwert. Der Start der Ruhephase beendet auch eine Abfrage der nicht belegten Buchsen.

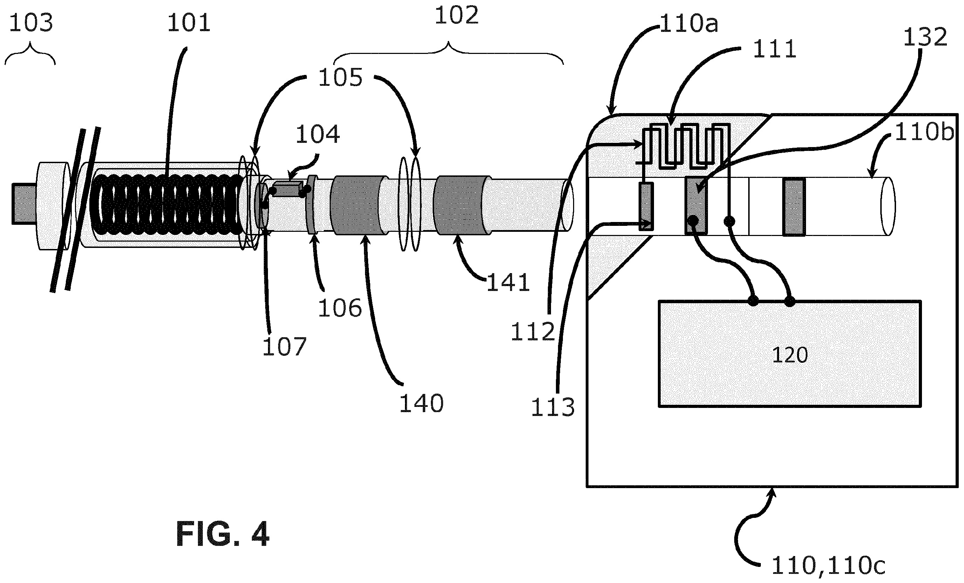

In einem weiteren Ausführungsbeispiel, das in Fig. 4 gezeigt ist, ist der RFID-Chip zusätzlich zu der galvanischen Verbindung mit dem Kontaktelement 106 galvanisch mit einem größeren metallischen Element (zum Beispiel in Form eines Metallrings) 107 kontaktiert, das kapazitiv an den elektrischen Leiter 101 der Elektrodenleitung gekoppelt ist und somit als Groundplane des RFID-Chips 104 dient. Bei diesem Ausführungsbeispiel ist ein therapeutisch genutztes Kontaktelement 132 der Buchse 110b an die Masse des Implantats 110 angeschlossen. Beim Einstecken des Steckers 102 der Elektrodenleitung 100 in die Buchse 110b wird ein therapeutisches Kontaktelement 140 der Elektrodenleitung 100 mit dem Kontaktelement 132 an der Buchse 110b verbunden, sodass über die kapazitive Kopplung mit dem mit dem Kontaktelement 140 verbundenen elektrischen Leiter 101 die Verbindung des RFID-Chips 104 mit der Masse des Implantats 110 erfolgt.

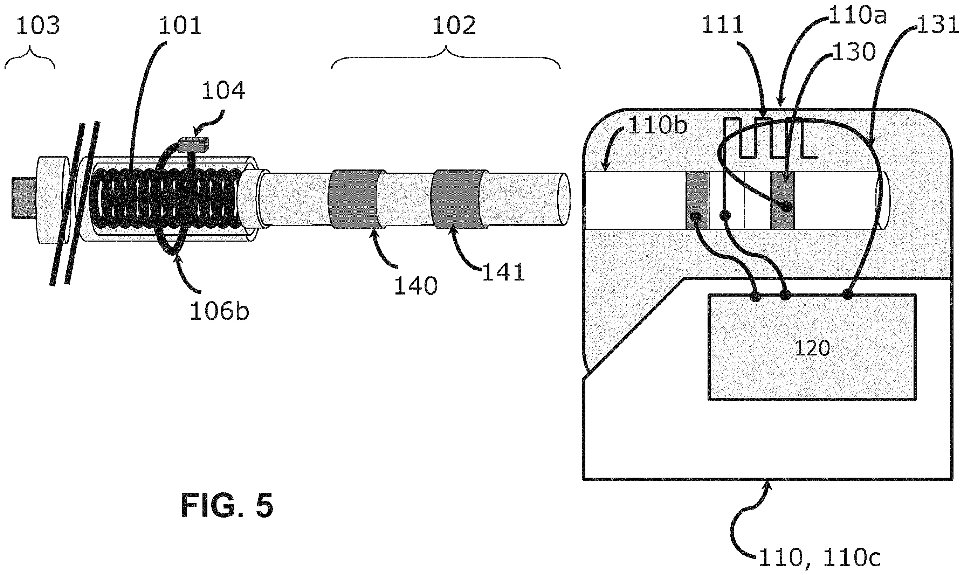

In dem in Fig. 5 gezeigten Ausführungsbeispiel ist eine weitere Alternative für die Kopplung des RFID-Chips 104 an einen elektrischen Leiter 101 der Elektrodenleitung 100 dargestellt. Der RFID-Chip 104 ist mit einer Leiterschleife 106b verbunden, welche kontaktlos an den elektrischen Leiter 101 koppelt. Weiter ist in dem Header 110a anstelle der zweiten Antenne 112 eine elektrische Leiterkomponente 131 (Leiterschleife) als elektromagnetisches Übertragungselement vorgesehen, welche elektromagnetisch oder kapazitiv mit der Kommunikationsantenne 111 gekoppelt ist. Die elektrische Leiterkomponente 131 ist an ihrem einen Ende mit der Steuereinrichtung 120 und an ihrem anderen Ende mit einem vorzugsweise auch therapeutisch genutzten Kontaktelement 130 der Buchse 110b verbunden. Beim Einstecken des Steckers 102 in die Buchse 110b erfolgt eine galvanische Verbindung mit einem entsprechenden Kontaktelement 140 des Steckers 102 und somit mit dem elektrischen Leiter 101 der Elektrodenleitung 100.

Die elektrische Leiterkomponente 131 verläuft nahe, vorzugsweise parallel zur Kommunikationsantenne 111, sodass sie bei der verwendeten UHF RFID-Frequenz möglichst optimal elektromagnetisch oder induktiv zur Kommunikationsantenne 111 koppelt. Besonders bevorzugt verläuft die Leiterkomponente 131 über eine Länge von mindestens 5 mm parallel zur Kommunikationsantenne 111. Vorzugsweise ist dabei der Abstand der Kommunikationsantenne 111 zu der Leiterkomponente 131 kleiner als 12 mm. Falls die Kommunikationsantenne 111 als Loop-Antenne gestaltet ist, ist es bevorzugt, dass sich die von der Kommunikationsantenne 111 und der Leiterkomponente 131 aufgespannten Flächen weitgehend überlagern. Anders ausgedrückt sollen sich der magnetische Fluss der Kommunikationsantenne 111 und der magnetische Fluss der Leiterkomponente 131 gegenseitig bestmöglich durchfluten. Vorzugsweise beträgt der magnetische Kopplungsfaktor zwischen der Kommunikationsantenne 111 und der Leiterkomponente 131 mindestens 0,1.

In einem Ausführungsbeispiel ist die Leiterkomponente 131 nahe der Eintrittsstelle in das elektrisch leitfähige Gehäuse 110c des Implantats 110 (beispielsweise näher als 20 mm) über einen nicht dargestellten Kondensator an das elektrisch leitfähige Gehäuse 110c angeschlossen, wobei der Kondensator als EMI-Schutz genutzt werden kann. Oben wurden bereits Möglichkeiten zur Dimensionierung des Kondensators erläutert.

Auch bei diesem Ausführungsbeispiel werden Elektrodenleitungen 100, die sich in unmittelbarer Umgebung des Headers 110a befinden, jedoch nicht mit dem aktiven Implantat kontaktiert sind, nicht ausgelesen. Dies erhöht die Zuverlässigkeit der Elektrodenleitungs-Identifikation.

In Abfragerichtung erfolgt der Weg des Abfragesignals somit von der Steuereinrichtung 120 über die Kommunikationsantenne 111, die elektrische Leiterkomponente 131, den elektrischen Leiter 101 der Elektrodenleitung 100, die Leiterschleife 106b zum RFID-Chip 104. Der Signalweg des Antwortsignals geht in die entgegengesetzte Richtung.

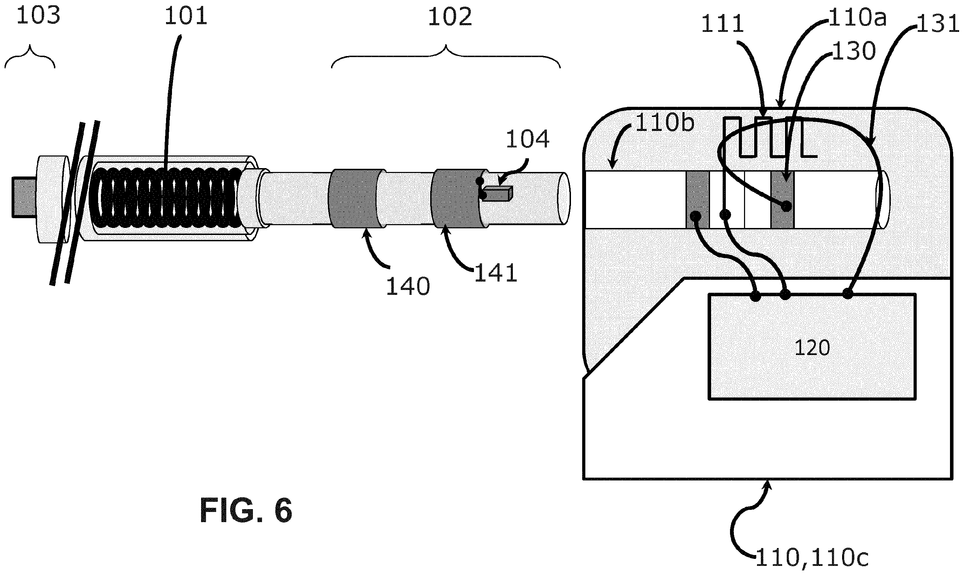

In dem in Fig. 6 dargestellten Ausführungsbeispiel ist eine direkte galvanische Kopplung zwischen RFID-Chip 104 und einem therapeutisch genutzten Kontaktelement 141 des Steckers 102 der Elektrodenleitung 100 vorgesehen. Implantatsseitig ist der Aufbau analog zu Fig. 5. Bevorzugt ist der RFID-Chip 104 mit dem Kontaktelement 141 verbunden, das einen Belag mit dem höchsten Induktivitäts- und/oder Widerstandswert der betreffenden Elektrodenleitung 100 aufweist. Dadurch wird der Einfluss der von dem Kontaktelement 141 aus gesehen distal liegenden elektrischen Leiter 101 auf die Übertragungseigenschaften des RFID-Chips minimiert.

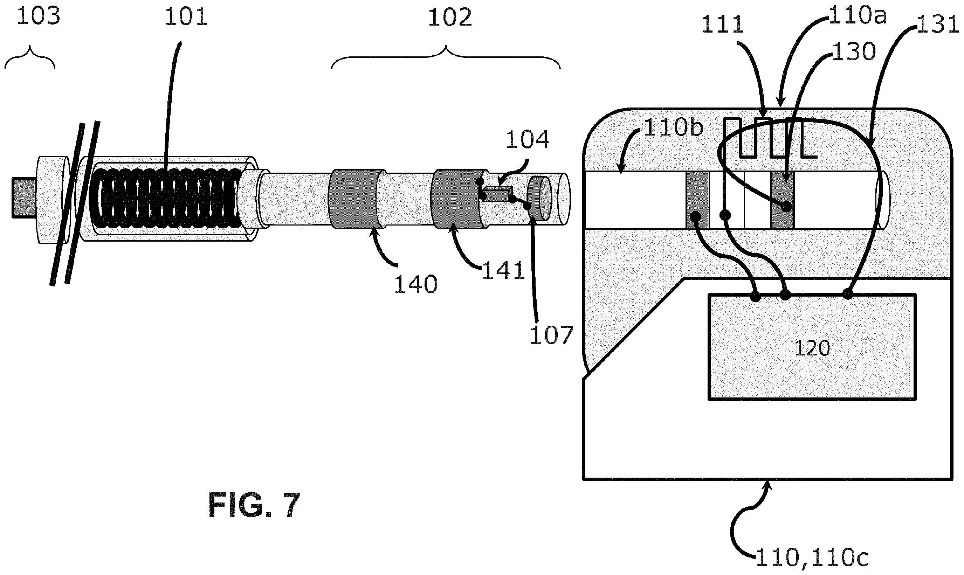

Bei dem in Fig. 7 gezeigten Ausführungsbeispiel ist der zweite Anschluss des RFID-Chips 104 mit einem größeren metallischen Element 107 (zum Beispiel in Form eines Metallrings) verbunden. Analog zu dem oben anhand von Fig. 4 erläuterten Ausführungsbeispiel wird hierdurch eine Groundplane für den RFID-Chip 104 realisiert.

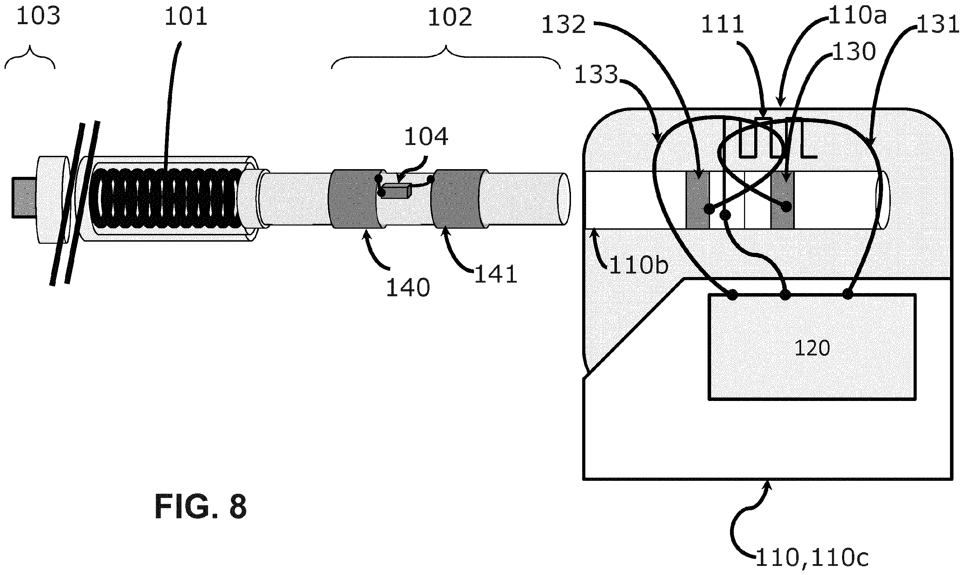

Das in Fig. 8 dargestellte Ausführungsbeispiel weist einen RFID-Chip 104 auf, der mit zwei therapeutisch genutzten Kontaktelementen 140, 141 galvanisch verbunden ist. Dieses Ausführungsbeispiel nutzt zwei Leiterkomponenten 131, 133 jeweils als elektromagnetisches Übertragungselement, das mit der Kommunikationsantenne 111 koppelt. In dieser Ausführungsform ist der RFID-Chip 104 mit beiden Enden an die Leiterkomponenten 131, 133 angebunden. Hierdurch gelingt eine Verbesserung der Kopplung gegenüber der Situation, dass das eine Ende des RFID-Chips an ein größeres Kontaktelement 107 angebunden ist, das als Groundplane des RFID-Chips 104 dient, da hierdurch mögliche Störungen aufgrund von Schwankungen des Groundplane-Potentials eliminiert werden.

In einer besonders bevorzugten Ausführungsform übermittelt die Steuereinrichtung 120 in den RFID-Chip 104 für kurze Zeit (bevorzugt für eine Zeit kürzer als 2 ms) durch eine galvanische Kopplung Energie und veranlasst den RFID-Chip 104 dadurch, seine Elektrodenleitungs-Informationen auszusenden. Diese werden über die Kommunikationsantenne 111 empfangen und an die Steuereinrichtung 120 weitergeleitet. Die Steuereinrichtung 120 kennt den Zeitpunkt, wann an welchen RFID-Chip 104 Energie geliefert wird und öffnet zeitlich passend das Empfangsfenster. Dadurch kann eine Zuordnung der entsprechenden Elektrodenleitung 100 zur jeweiligen Buchse 110b bzw. den entsprechenden Kanal erfolgen.

Der Energiepuls wird, falls das Implantat als Herzschrittmacher ausgeführt ist, vorzugsweise über einen Pace geliefert bzw. davon abgeleitet. Der RFID-Chip ist dabei so gestaltet, dass dessen Energie-Verbrauch geringer ist als 1 % der Paceenergie. Der Innenwiderstand des RFID-Chips 104 ist so dimensioniert, dass er, wenn er zwischen den Kontaktelementen 140, 141 des Steckers 102 gemessen wird, mindestens 1 kΩ beträgt. Besonders bevorzugt ist der Innenwiderstand größer als 1MΩ.

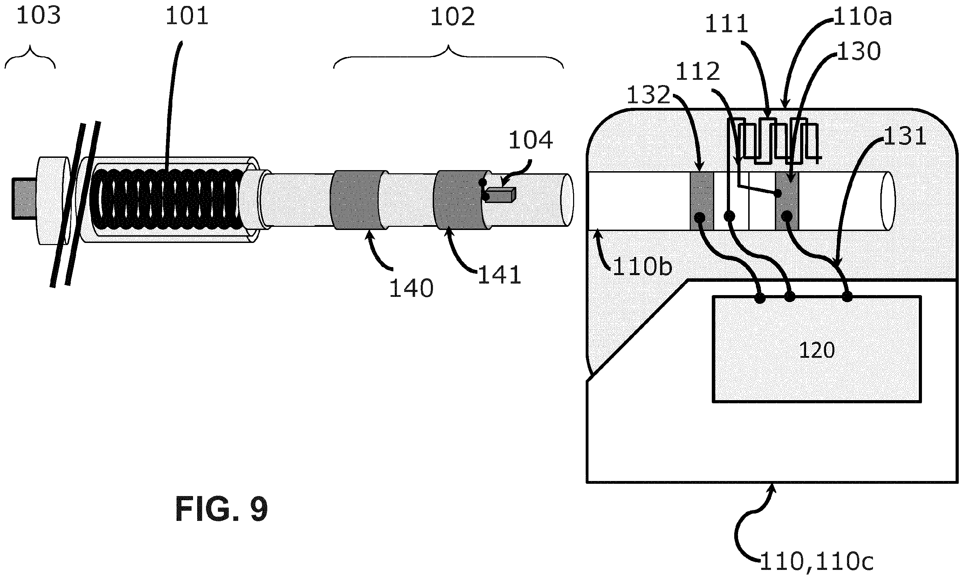

Das in Fig. 9 dargestellte Ausführungsbeispiel ähnelt dem Ausführungsbeispiel gemäß Fig. 1, wobei bei der in Fig. 9 dargestellten Variante der RFID-Chip direkt galvanisch mit einem therapeutischen Kontaktelement 141 des Steckers 102 der Elektrodenleitung 100 verbunden ist. Entsprechend ist die Antenne 112 mit einem therapeutisch genutzten Kontaktelement 130 der Buchse 110b verbunden.

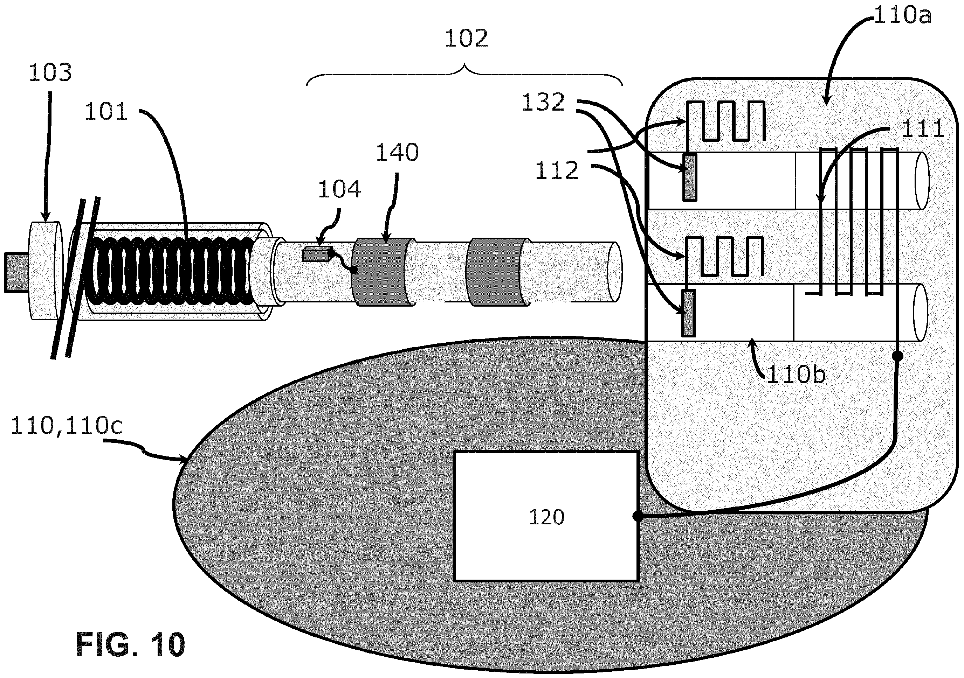

Das in Fig. 10 dargestellte Ausführungsbeispiel bezieht sich analog zu Fig. 3 auf eine Gestaltung, bei der jeder Buchse 110b eine Antenne 112 zugeordnet ist, während die einzige Kommunikationsantenne 111 so gestaltet ist, dass sie beide Buchsen 110b überdeckt. Das Ausführungsbeispiel gemäß Fig. 10 unterscheidet sich von dem Ausführungsbeispiel gemäß Fig. 3 darin, dass der RFID-Chip 104 direkt galvanisch mit einem therapeutischen Kontaktelement 140 des Steckers 102 der Elektrodenleitung 100 verbunden ist. Entsprechend ist die jeder Buchse zugeordnete zweite Antenne 112 galvanisch mit einem therapeutisch genutzten Kontaktelement 132 der Buchse 110b verbunden.

Wie bereits oben erläutert wurde, kann der RFID-Chip 104 aktiv oder passiv gestaltet sein. Im Falle einer passiven Gestaltung des RFID-Chips 104 können oben dargestellte Verfahren zur Aktivierung des RFID-Chips 104 verwendet werden.

Das Implantat weist in einem bevorzugten Ausführungsbeispiel eine nicht dargestellte Detektionseinheit auf, welche die Kontaktierung einer Elektrodenleitung 100 mit dem Implantat 110 bzw. das ordnungsgemäße Einstecken eines Steckers 102 einer Elektrodenleitung 100 in eine Buchse 110b detektiert. Oben wurde bereits erläutert, über welche Verfahren eine derartige Detektionseinheit, die mit der Steuereinrichtung 120 verbunden ist, feststellen kann, dass eine Kontaktierung bzw. ein ordnungsgemäßes Einstecken des Steckers 102 erfolgt ist. Eine derartige Detektionseinheit kann erfindungsgemäß bei allen oben erläuterten Ausführungsbeispielen der Implantate eingesetzt werden.

Durch die vorliegende Erfindung werden elektrodenspezifische Informationen aller mit einem Implantat 110 gekoppelten Elektrodenleitungen 100 ausgelesen, ohne den therapeutischen Pfad durch eine serielle galvanische Kopplung oder zusätzliche elektrische Komponenten zu gefährden. Der RFID-Chip 104 wird erst durch die Kontaktierung mit dem Implantat 110 mit einer Kommunikationsantenne 111 kontaktiert. Die Zuordnung der Elektrodenleitung 100 zur jeweiligen Buchse 110b bzw. Kanal erfolgt über das gezielte Ansprechen der Buchsen/Kanäle durch das aktive Implantat 110. Der Leistungsbedarf zur Identifikation ist gegenüber einer Auslesung eines RFID-Inlays (mit Antenne) über eine Sendeantenne am aktiven Implantat deutlich reduziert. Es sind zudem keine Vorkehrungen erforderlich, um die Funktion der EMI-Filter für das Auslesen zu umgehen. Vorteilhaft ist ferner, dass der RFID-Chip kleinbauend ist und beispielsweise einen deutlich geringeren Platzbedarf aufweist als ein RFID-Inlay (mit Antenne). Die Anordnung des Übertragungselements im Header 110a führt dazu, dass das Übertragungselement lediglich geringen mechanischen Belastungen ausgesetzt ist.

### Bezugszeichenliste

- 100: Elektrodenleitung
- 101: elektrischer Leiter
- 102: Stecker
- 103: distales Ende der Elektrodenleitung
- 104: RFID-Chip
- 105: Dichtlippen
- 106: Chip-Kontaktelement
- 106a: metallisierte Dichtlippen
- 106b: Leiterschleife
- 107: metallisches Element
- 110: Implantat
- 110a: Header des Implantats
- 110b: Buchse
- 110c: Gehäuse
- 111: Kommunikationsantenne
- 112: zweite Antenne
- 113: Kontaktelement
- 120: Steuereinrichtung
- 130, 132: Kontaktelement der Buchse 110b
- 131, 133: Leiterkomponente
- 140, 141: Kontaktelement des Steckers 102 der Elektrodenleitung 100

## Patentansprüche

1. Implantat (110) mit einem hermetisch dicht abgeschlossenem Gehäuse (110c), wobei in dem Gehäuse (110c) eine Steuereinrichtung (120) angeordnet ist, und mit einem an dem Gehäuse (110c) befestigten Header (110a) aufweisend mindestens eine Buchse (110b) zur Verbindung mit einem Stecker (102) einer Elektrodenleitung (100) und eine Kommunikationsantenne (111), welche elektrisch mit der Steuereinrichtung (120) verbunden ist, **dadurch gekennzeichnet, dass** der Header (110a) im Bereich der mindestens einen Buchse (110b) mindestens ein elektromagnetisches Übertragungselement (112, 131, 133) aufweist, das elektrisch mit einem Kontaktelement, welches an der Innenwand der mindestens einen Buchse (110b) vorgesehen ist verbunden ist, wobei das elektromagnetische Übertragungselement (112, 131, 133) elektromagnetisch oder induktiv mit der Kommunikationsantenne (111) gekoppelt ist.

2. Implantat nach Anspruch 1, **dadurch gekennzeichnet, dass** das elektromagnetische Übertragungselement als Antenne (112) oder elektrische Leiterkomponente (131, 133) ausgebildet ist.

3. Implantat nach Anspruch 2, **dadurch gekennzeichnet, dass** die elektrische Leiterkomponente (131) über einen Kondensator an das Gehäuse (110c) des Implantats (110) angeschlossen ist, das elektrisch leitfähig ausgebildet ist.

4. Implantat nach einem der Ansprüche 1 bis 3, **dadurch gekennzeichnet, dass** das Implantat eine Vielzahl von Buchsen (110b) im Header (110a) aufweist und die Anzahl der elektromagnetischen Übertragungselemente (112, 131, 133) mit der Anzahl der Buchsen (110b) korrespondiert, wobei eine einzige Kommunikationsantenne (111) vorgesehen ist, die mit allen im und/oder am Header (110a) angeordneten elektromagnetischen Übertragungselementen (112, 131, 133) elektromagnetisch oder induktiv gekoppelt ist.

5. Implantat nach einem der Ansprüche 1 bis 4, **dadurch gekennzeichnet, dass** die Steuereinrichtung (120) derart eingerichtet ist, dass sie nach dem Auftreten eines vorbestimmten Ereignisses für jede mit einer Elektrodenleitung verbundene Buchse (110b) ein separates Abfragesignal erzeugt.

6. Implantat nach einem der Ansprüche 1 bis 5, **dadurch gekennzeichnet, dass** das Implantat eine Detektionseinheit aufweist, welche vorzugsweise das Anschließen der Elektrodenleitung (100) an das Implantat (110) detektiert.

7. Verfahren zur Identifikation einer Elektrodenleitung (100) mittels eines Implantats (110) nach einem der Ansprüche 1 bis 6,
wobei die Elektrodenleitung (100) einen Stecker (102) zur Verbindung mit einem Implantat (110), mindestens einen elektrischen Leiter (101) und einen den mindestens einen elektrischen Leiter (101) isolierenden Isolationsschlauch (130) aufweist, wobei ein hermetisch dicht abgeschlossener RFID-Chip (104) in den Isolationsschlauch (130) und/oder in den Stecker (102) oder in einen isolierenden Körper eines mit dem Isolationsschlauch (130) oder den Stecker (102) vorzugsweise formschlüssig verbindbaren, separaten Zusatzteil eingebettet ist, wobei der RFID-Chip (104) elektrisch mit einer Leiterschleife (106b) oder mit mindestens einem an dem Stecker (102) angeordneten Kontaktelement (106, 106a, 140, 141) verbunden ist; und wobei das Implantat (110) mit der Elektrodenleitung (100) verbunden ist, umfassend die folgenden Schritte:
- Erzeugen eines elektromagnetischen Abfragesignals durch die Steuereinrichtung (120) und Weiterleiten an die mit der Steuereinrichtung verbundene Kommunikationsantenne (111),
- Senden des elektromagnetischen Abfragesignals durch die Kommunikationsantenne (111),
- Empfangen des Abfragesignals durch das Übertragungselement (112, 131, 133) mittels elektromagnetischer oder induktiver Kopplung,
- Weiterleiten des empfangenen Abfragesignals an den RFID-Chip (104),
- Verarbeiten des empfangenen Abfragesignals durch den RFID-Chip (104),
- Erzeugen eines entsprechenden elektromagnetischen Antwortsignals durch den RFID-Chip (104) und Übertragen des Antwortsignals an das Übertragungselement (112, 131, 133),
- Empfangen des Antwortsignals durch die Kommunikationsantenne (111) des Implantats mittels elektromagnetischer oder induktiver Kopplung mit dem Übertragungselement (112, 131, 133) und Weiterleiten an die Steuereinrichtung (120) sowie
- Verarbeiten des weitergeleiteten empfangenen Antwortsignals in der Steuereinrichtung (120).

8. Verfahren nach Anspruch 7, **dadurch gekennzeichnet, dass** die Erzeugung des Abfragesignals durch die Steuereinrichtung (120) nach dem Auftreten eines vorbestimmten Ereignisses, vorzugsweise nach dem Anschließen einer Elektrodenleitung (100) an das Implantat (110) erfolgt.

9. Verfahren nach einem der Ansprüche 7 bis 8, **dadurch gekennzeichnet, dass** dann, wenn zwei oder mehr als zwei Elektrodenleitungen (100) an das Implantat (110) angeschlossen sind, wobei von diesen Elektrodenleitungen (100) eine Elektrodenleitung zuletzt an das Implantat (110) angeschlossen ist, das Verfahren zur Identifikation für alle angeschlossenen Elektrodenleitungen (100) nacheinander durchgeführt wird, und dass das jeweilige Antwortsignal mit den bereits in einer Speichereinrichtung des Implantats (110) gespeicherten Informationen zu den Elektrodenleitungen (100) verglichen und basierend auf dem Ergebnis des Vergleichs eine Zuordnung der Informationen der zuletzt angeschlossenen Elektrodenleitung (100) zu der jeweiligen Buchse des Implantats (110) erfolgt.

## Claims

1. An implant (110) with a hermetically tightly sealed housing (110c), the housing (110c) having a controller (120) arranged in it, and with a header (110a) that is fastened to the housing (110c) and that has at least one socket (110b) for connection with a plug (102) of an electrode lead (100) and that has a communication antenna (111) that is electrically connected with the controller (120), **characterized in that** the header (110a) has, in the area of the at least one socket (110b), at least one electromagnetic transmission element (112, 131, 133) that is electrically connected with a contact element provided on the inner wall of the at least one socket (110b), the electromagnetic transmission element (112, 131, 133) being electromagnetically or inductively coupled with the communication antenna (111).

2. An implant according to claim 1, **characterized in that** the electromagnetic transmission element is in the form of an antenna (112) or electrical conductor component (131, 133).

3. An implant according to claim 2, **characterized in that** the electrical conductor component (131) is connected to the housing (110c) of the implant (110) through a capacitor, the housing being electrically conductive.

4. An implant according to any one of the claims 1 through 3, **characterized in that** the implant has multiple sockets (110b) in the header (110a) and **in that** the number of the electromagnetic transmission elements (112, 131, 133) corresponds to the number of the sockets (110b), a single communication antenna (111) being provided, which is electromagnetically or inductively coupled with all electromagnetic transmission elements (112, 131, 133) arranged in and/or on the header (110a).

5. An implant according to any one of the claims 1 through 4, **characterized in that** the controller (120) is set up so that it produces a separate interrogation signal after the occurrence of a predetermined event for every socket (110b) that is connected with an electrode lead.

6. An implant according to any one of claims 1 through 5, **characterized in that** the implant has a detection unit, which preferably detects the connection of the electrode lead (100) to the implant (110).

7. A process to identify an electrode lead (100) by means of an implant (110), according to any one of the claims 1 through 6,
the electrode lead (100) having a plug (102) for connection with an implant (110), at least one electrical conductor (101), and an insulating tube (130) that insulates the at least one electrical conductor (101), a hermetically tightly sealed RFID chip (104) being embedded in the insulating tube (130) and/or in the plug (102) or in an insulating body of a separate accessory that is connectable, preferably connectable in a form-fit manner, with the insulating tube (130) or the plug (102), the RFID chip (104) being electrically connected with a conductor loop (106b) or with at least one contact element (106, 106a, 140, 141) arranged on the plug (102); and the implant (110) being connected with the electrode lead (100), this process comprising the following steps:
- The controller (120) producing an electromagnetic interrogation signal and forwarding it to the communication antenna (111), which is connected with the controller;
- The communication antenna (111) transmitting the electromagnetic interrogation signal;
- The transmission element (112, 131, 133) receiving the interrogation signal by means of electromagnetic or inductive coupling;
- The received interrogation signal being forwarded to the RFID chip (104);
- The RFID chip (104) processing the received interrogation signal;
- The RFID chip (104) producing a corresponding electromagnetic response signal and transferring the response signal to the transmission element (112, 131, 133);
- The communication antenna (111) of the implant receiving the response signal by means of electromagnetic or inductive coupling with the transmission element (112, 131, 133), and forwarding it to the controller (120); and
- The received forwarded response signal being processed in the controller (120).

8. The process according to claim 7, **characterized in that** the controller (120) produces the interrogation signal after the occurrence of a predetermined event, preferably after the connection of an electrode lead (100) to the implant (110).

9. The process according to any one of claims 7 through 8, **characterized in that** when two or more than two electrode leads (100) are connected to the implant (110), if one of these electrode leads (100) was connected to the implant (110) last, the identification process is carried out for all connected electrode leads (100) one after the other, and **in that** the respective response signal is compared with the information on the electrode leads (100) that is already stored in a storage device of the implant (110) and, based on the result of the comparison, the information of the last connected electrode lead (100) is associated with the respective socket of the implant (110).

## Revendications

1. Implant (110) avec un boîtier (110c) fermé hermétiquement de manière étanche, où un dispositif de commande (120) est disposé dans le boîtier (110c), et avec un collecteur (110a) fixé sur le boîtier (110c) présentant au moins une douille (110b) pour le raccordement avec une prise (102) d'une ligne d'électrode (100) et une antenne de communication (111), laquelle est reliée électriquement avec le dispositif de commande (120), **caractérisé en ce que** le collecteur (110a) présente au moins un élément de transmission électromagnétique (112, 131, 133) dans la région de l'au moins une douille (110b), qui est relié électriquement avec un élément de contact, lequel est prévu sur la paroi intérieure de l'au moins une douille (110b), où l'élément de transmission électromagnétique (112, 131, 133) est couplé de manière électromagnétique ou inductive avec l'antenne de communication (111).

2. Implant selon la revendication 1, **caractérisé en ce que** l'élément de transmission électromagnétique est conçu sous forme d'antenne (112) ou de composant conducteur électrique (131, 133).

3. Implant selon la revendication 2, **caractérisé en ce que** le composant conducteur électrique (131) est raccordé au boîtier (110c) de l'implant (110) qui est conçu comme étant conducteur électriquement, par le biais d'un condensateur.

4. Implant selon l'une des revendications 1 à 3, **caractérisé en ce que** l'implant présente une multiplicité de douilles (110b) dans le collecteur (110a) et le nombre des éléments de transmission électromagnétiques (112, 131, 133) correspond au nombre de douilles (110b), où une seule antenne de communication (111) est prévue qui est couplée de manière électromagnétique ou inductive avec tous les éléments de transmission électromagnétiques (112, 131, 133) disposés dans et/ou sur le collecteur (110a).

5. Implant selon l'une des revendications 1 à 3, **caractérisé en ce que** le dispositif de commande (120) est conçu de telle manière qu'il génère un signal d'interrogation séparé pour chacune des douilles (110b) reliées avec la ligne d'électrode après la survenue d'un événement prédéterminé.

6. Implant selon l'une des revendications 1 à 5, **caractérisé en ce que** l'implant présente une unité de détection laquelle détecte de préférence le branchement de la ligne d'électrode (100) sur l'implant (110).

7. Procédé d'identification d'une ligne d'électrode (110) au moyen d'un implant (110) selon l'une des revendications 1 à 6,
dans lequel la ligne d'électrode (100) présente une prise (102) pour la connexion avec un implant (110), au moins un conducteur électrique (101) et un tuyau d'isolation (130) isolant l'au moins un conducteur électrique (101), où une puce RFID (104) fermée hermétiquement de manière étanche est incorporée dans le tuyau d'isolation (130) et/ou dans la prise (102) ou dans un corps isolant d'une partie complémentaire séparée, pouvant être reliée de préférence par complémentarité des formes avec le tuyau d'isolation (130) ou la prise (102), où la puce RFID (104) est reliée électriquement avec une boucle conductrice (106b) ou avec au moins un élément de contact (106, 106a, 140, 141) disposé sur au moins une prise (102) ; et où l'implant (110) est relié avec la ligne d'électrode (100), comprenant les étapes suivantes :
- de génération d'un signal d'interrogation électromagnétique par le dispositif de commande (120) et de transmission à l'antenne de communication (111) reliée avec le dispositif de commande,
- d'envoi du signal d'interrogation électromagnétique par l'antenne de communication (111),
- de réception du signal d'interrogation par l'élément de transmission (112, 131, 133) au moyen d'un couplage électromagnétique ou inductif,
- de transmission du signal d'interrogation reçu à la puce RFID (104),
- de traitement du signal d'interrogation reçu par la puce RFID (104),
- de génération d'un signal de réponse électromagnétique correspondant par la puce RFID (104) et de transmission du signal de réponse à l'élément de transmission (112, 131, 133),
- de réception du signal de réponse par l'antenne de communication (111) de l'implant au moyen d'un couplage électromagnétique ou inductif avec l'élément de transmission (112, 131, 133) et de transfert vers le dispositif de commande (120), ainsi que
- de traitement du signal de réponse reçu transféré dans le dispositif de commande (120).

8. Procédé selon la revendication 7, **caractérisé en ce que** la génération du signal d'interrogation a lieu par le dispositif de commande (120) après la survenue de l'événement prédéfini, de préférence, après le raccordement d'une ligne d'électrode (100) sur l'implant (110).

9. Procédé selon l'une des revendications 7 à 8, **caractérisé en ce que,** lorsque deux ou plus de deux lignes d'électrode (100) sont raccordées sur l'implant (110), où parmi ces lignes d'électrode (100) une ligne d'électrode est raccordée en dernier lieu sur l'implant (110), le procédé est exécuté pour l'identification de toutes les lignes d'électrodes (100) raccordées les unes après les autres et que le signal de réponse respectif est comparé avec les informations concernant les lignes d'électrodes (100) stockées déjà stockées dans un dispositif de stockage de l'implant (110) et en se basant sur le résultat de la comparaison, une association des informations de la ligne d'électrode (100) raccordée en dernier lieu a lieu avec la douille respective de l'implant (110).
